# EUROPEAN PATENT APPLICATION

(11) **EP 4 843 585 A1**
(43) Date of publication of application: **25.11.2026**
(21) Application number: 25178495.5
(22) Date of filing: 23.05.2025
(51) Int. Cl.: A61K 40/31, C07K 14/725, C07K 16/28

(54) **CHIMERIC ANTIGEN RECEPTOR COMPRISING HINGE AND TRANSMEMBRANE DR5 DOMAIN FOR IMMUNOTHERAPY**

(71) Applicant: Kemijski Institut, 1000 Ljubljana (SI)
(72) Inventor: JERALA, Roman, 1000 Ljubljana (SI); FINK, Tina, 1000 Ljubljana (SI); VOKAC KRIZAJ, Hana, 1000 Ljubljana (SI)
(74) Representative: Weickmann & Weickmann PartmbB

(57) **Abstract**

Chimeric antigen receptor (CAR-T) cell therapy is an advanced cancer treatment, particularly for B-cell leukemia and lymphoma, as well as autoimmune diseases. However, challenges such as toxicity, limited tumor infiltration, and antigen escape hinder its full potential. This invention relates to an innovative modification to second-generation CARs by replacing the conventional CD8α transmembrane (TMD) and hinge domain with the Death Receptor 5 (DR5) transmembrane and ectodomain. This structural enhancement improves tumor-killing efficacy and activation, leading to superior tumor or other target cell clearance.

The DR5-based CAR retains the core components of second-generation CARs, including a targeting domain and intracellular costimulatory (4-1 BB or CD28) and CD3ζ signaling domains, while incorporating the DR5 hinge and transmembrane domain. This alteration enhances oligomerization and activation, strengthening immune response efficiency without constitutive activity in the absence of an antigen. In vitro and in vivo studies demonstrated improved tumor eradication and increased expression of the activation marker CD69 of DR5-based CAR T cells compared to conventional CAR constructs. Further validation using other target BCMA-directed CARs confirmed the broad applicability of this modification.

This invention offers a major advancement in CAR T cell therapy, extending efficient treatment to cells with low antigen density.

## Description

### FIELD OF THE INVENTION

Chimeric antigen receptor (CAR-T) cell therapy represents a highly efficient option for the treatment of cancer or other diseases where the elimination of a specific cell type is desired. This type of therapy has made incredible advances in treating B cell leukemia and lymphoma, autoimmune diseases and is aimed to address solid tumors in the future. Barriers to the effectiveness of this therapy lie in its potential side effects, limited tumor infiltration, and antigen escape. This invention improves the second-generation CAR by switching the conventional Transmembrane domain (TMD) CD8α and hinge domain CD8α with a DR5 TMD and hinge (ED) as the hinge domain from the Death receptor 5. With this approach, tumor-killing and activation are improved, providing better tumor clearance overall.

### BACKGROUND

CAR-T therapy is a personalized cell-based therapy where patients' own T cells are modified with CAR construct for them to recognize and eliminate cancer cells. CAR has a single-chain variable fragment (scFv) that recognizes and binds a tumor-associated target, without the need for an MHC receptor resulting in T-cell activation. CAR takes advantage of similar functional modules that make up the T cell receptor (TCR) and its costimulatory receptors mimicking its function (June and Sadelain 2018). The second-generation CAR which was FDA-approved in 2017, has a scFv and a hinge region on the outside of the T cell (Awasthi et al. 2023). The hinge is usually derived from CD8, CD28, IgG1, or IgG4. The standard of care CAR, we will be comparing our invention to, has a CD8α hinge derived from a T-cell costimulatory receptor. Adjacent to it is the CD8α TMD derived from the same protein although CD3ζ, CD4, or CD28 have also been used in the past (Jayaraman et al. 2020). TMD is followed by intracellular signaling domains such as the costimulatory domain 4-1BB and the activation domain CD3ζ.

Transmembrane domains are essential structural regions of membrane-bound proteins that anchor receptors in the lipid bilayer and facilitate communication between extracellular and intracellular environments. In the TCR-CD3 complex the TMD CD3ζ, which is also present in the first-generation CAR, plays an essential role in receptor assembly and function in physiological T cells. The CD3ζ TM domain is particularly critical for stabilizing interactions with other CD3 subunits, ensuring the integrity of the TCR-CD3 complex. It facilitates dimerization and supports interactions with endogenous TCRα and TCRβ chains, enabling both cis and trans-signaling mechanisms. These mechanisms are mediated by ionic interactions between transmembrane domains, allowing the cytoplasmic signaling units of the TCR-CD3 complex to coordinate adaptive immune responses (Bridgeman et al., 2010).

Studies on the first-generation chimeric antigen receptors (CARs) have investigated how variations in the hinge and transmembrane (TM) domains influence CAR expression and functional activity. Fujiwara et al. (2020) explored this by examining CAR constructs with hinge and TM regions derived from CD3ζ, CD4, CD8α, and CD28. They determined that CARs incorporating TMDs from CD8α or CD28 exhibited higher expression levels on the T-cell surface compared to CD3ζ-based CARs. Furthermore, CARs with CD28-derived hinges demonstrated superior signaling compared to those with CD8α-derived hinges. The study concluded that the hinge domain regulates the signaling threshold of CARs, while the transmembrane domain governs the magnitude of signaling, as reflected by CAR expression levels (Fujiwara et al., 2020).

In another study, Li et al. (2023) modified the hinge and TM domains of a second-generation glypican-1 (GPC1) CAR to enhance CAR-T cell functionality, particularly against tumors with low antigen density-a significant challenge in solid tumors like pancreatic cancer. The researchers compared combinations of IgG4 and CD8 hinges with CD8 and CD28 transmembrane domains. Notably, the combination of an IgG4 hinge and CD28 TM domain significantly improved CAR-T cell functionality. This configuration enhanced the dimerization of the CARs, boosting T-cell signaling capacity and improving tumor-killing efficiency (Li et al. 2023).

Further, WO 2017/062820 discloses activity-enhanced TCRs comprising the transmembrane domain of TNFR superfamily members 16 or 19 (TNFRSF-16 or TNFRSF-19), whereby the transmembrane domain of these receptors comprises a conserved amino acid feature combination which aligns with features of the transmembrane domain of CD4 and CD8, but which is not ubiquitous throughout the TNFR-superfamily, and which is absent in DR5. It is further disclosed therein that the significance of this feature combination lies in the formation of a unique secondary structure within the plasma membrane of the cell, allowing respective CAR constructs optimal signal transduction and T cell activation.

Interestingly, the transmembrane domains disclosed in WO 2017/06820, which exhibit the conserved sequence motif, are dimerizing domains. In contrast, apoptotic members of the tumor necrosis factor receptor superfamily predominantly depend on higher-order oligomerization involving trimerization and hexamerization, as exemplified for FASL/FAS or TRAIL/DR5 signal transduction (Pan et al., 2019), where hexamerization involves the formation of dimers of trimers - a process critical for the efficient induction of programmed cell death (PCD).

The inventors were surprisingly able to demonstrate that the TMD ED of the DR5 receptor, which naturally mediates trimer-dimer interactions of the receptor, is particularly well-suited to improve CAR constructs by enhancing their clustering capacity. This enables the CAR molecule to form a stable signaling clusters and a stronger immunological synapse, resulting in more potent T-cell activation and enhanced tumor cell killing. Moreover, it provides the CART T cell with increased sensitivity towards cancer cells with a lower antigen density, due to efficient oligomer formation upon binding to target cells.

### SUMMARY OF THE INVENTION

Since dimerization is a crucial feature of the hinge and TMD, facilitating CAR clustering and oligomerization, this invention is specifically designed to enhance this process. TMD DR5 ED was selected from Death receptor 5. It belongs to the death receptor subfamily of the tumor necrosis factor receptor superfamily (TNFRSF) and its task is to mediate cell death and inflammation in many cell types. For this invention only a part of the ectodomain and the transmembrane spanning α-helix has been selected, without catalytic parts such as the ligand binding domain or death domain had been introduced to the final CAR construct. Thereby keeping the complex oligomer formation domains (dimer-trimer interactions), which are specific for at least the PCD inducing members of this family of receptors, while omitting the receptor function and downstream activation of caspase-8 which would potentially lead to apoptosis induction. While it has been assumed that oligomerization may lead to the constitutive tonic activity of the CAR, which is not desired, we surprisingly found that this construction principle did not trigger constitutive activity. Moreover, it enabled more potent activation compared to the conventional constructs based on CD8 hinge and transmembrane domain (TMD), resulting in improved therapeutic efficiency.

The invention is a new type of chimeric antigen receptor (CAR) that incorporates a new hinge and transmembrane domain into the second-generation CAR. The novelty is not only the introduction of a completely new domain used in CAR T therapy but also the use of oligomerization to achieve higher activation of CAR T cells. The DR5 domain may be applicable across different CAR constructs with different targeting domains targeting various tumor antigens. Using just the TMD is sufficient to get a well-functioning CAR however the addition of the hinge domain in combination with the TMD works best. There is also the fact that this CAR shows a higher activation marker presentation than the conventional CAR explaining the higher tumor clearance.

This invention represents a notable advancement in CAR-based cell therapy by enabling effective targeting of cells even at low antigen density - a known limitation of current therapies that contributes to cancer immune escape, particularly in solid tumors. By demonstrating efficacy across a range of antigens, including CD19, HER2, and BCMA, the invention broadens the applicability of CAR technology and supports its use in diverse therapeutic contexts, including solid and hematological proliferative disorders as well as autoimmune diseases, through improved functional sensitivity and adaptability.

Thus, improved CARs comprising DR5 ectodomain and/or DR5 TMD, nucleic acids encoding such improved CARs and therapeutic cells comprising the CARs and/or nucleic acids encoding such improved CARs are provided herein alongside their therapeutic use.

### BRIEF DESCRIPTION OF THE FIGURES

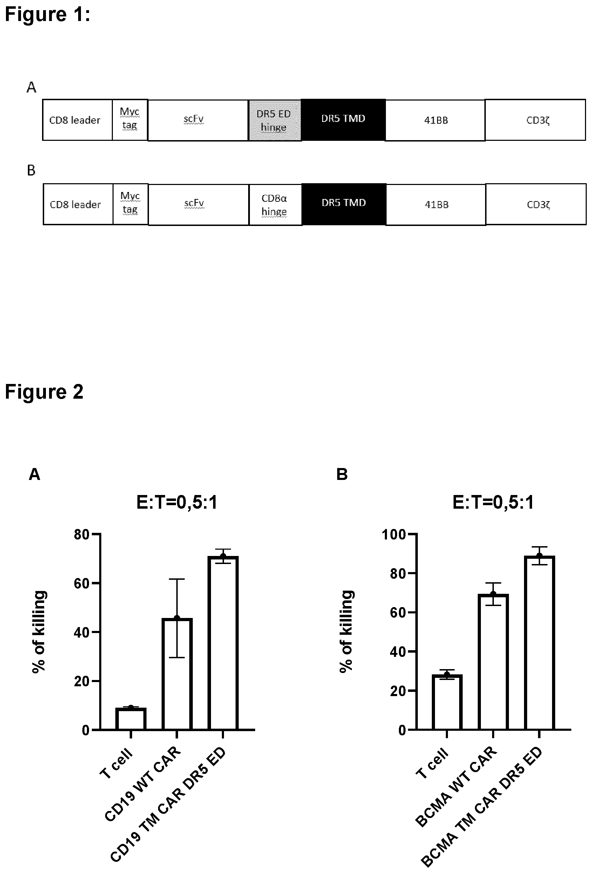

**Figure 1****: Scheme of CAR constructs with DR5 hinge and transmembrane domain**
   Scheme of CAR constructs incorporating DR5 hinge and transmembrane domains **(A)** or only DR5-derived transmembrane domain in combination with CD8α hinge domain **(B).** Antigen targeting is realized by selecting a suitable scFv sequence that is specific to the target antigen and different types of target cells.
**Figure 2****: Killing efficacy of DR5-based CAR T cells against different targets**
   Comparison of tumor cell killing ability between WT CAR (second-generation CAR with a CD8 TMD of SEQ ID NO: 43 and 44) and TM DR5 ED CAR (CAR of SEQ ID NO: 5 to 10 with DR5 hinge and TMD presented in this invention) after 24h at an effector-to-target ratio of 1:1.
   Targeting CD19 on RAJI cells **(A)** and targeting BCMA on RAJI cells **(B).**
**Figure 3****: Cytokine secretion of DR5-based CAR T cells**
   Human IL-2, IFN-γ, and Granzyme B levels in supernatants were collected from co-cultures of CAR T cells with tumor cells at an effector-to-target ratio of 1:1 after 24 hours. Targeting CD19 on RAJI cells.
**Figure 4****: Killing efficacy of DR5-based CAR constructs against tumor cells with different antigen densities**
   Killing efficacy of CAR T cells expressing TM DR5 ED CAR cocultured with tumor cells with different antigen densities. Tumor cells with lower antigen densities are K562-Fluc cells, one line with an MFI of 15000 (LOW) and the other 86000 for CD19 (MID). Raji-Fluc cells express the highest antigen density with an MFI of 300000 (HIGH). The effector (CAR-T) and target cells (K562, Raji-Fluc) are in a ratio of 1:1, measured after 24h.

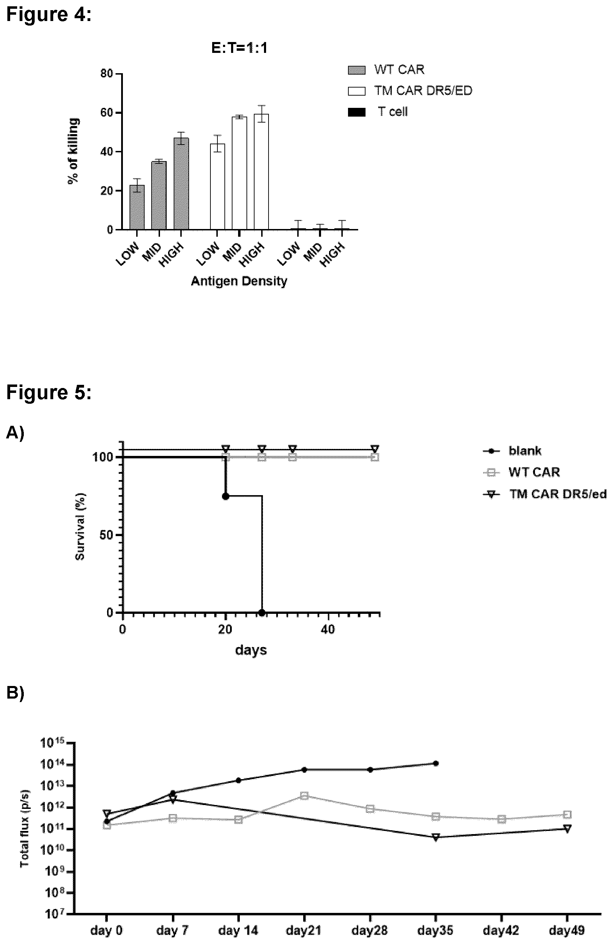

**Figure 5****: In vivo experimental animal survival and tumor bioluminescence analysis by treatment with DR5-based CAR T cells**
   The in vivo study was performed with NGS mice (NOD scid gamma) via intravenous injection of 1 × 10^6 RAJI-FLuc cells per mouse. After tumor establishment, CAR T cells (1 × 10^6 per mouse) were administered intravenously to evaluate the therapeutic efficacy. At day 28 all mice received 1 × 10^6 RAJI-FLuc cells per mouse again as a rechallenge. (A) The survival curve shows the percentage of animal survival over time after CAR T cell injection. (B) Total flux (p/s) over time, reflecting tumor burden through bioluminescent imaging of RAJI-FLuc cells.

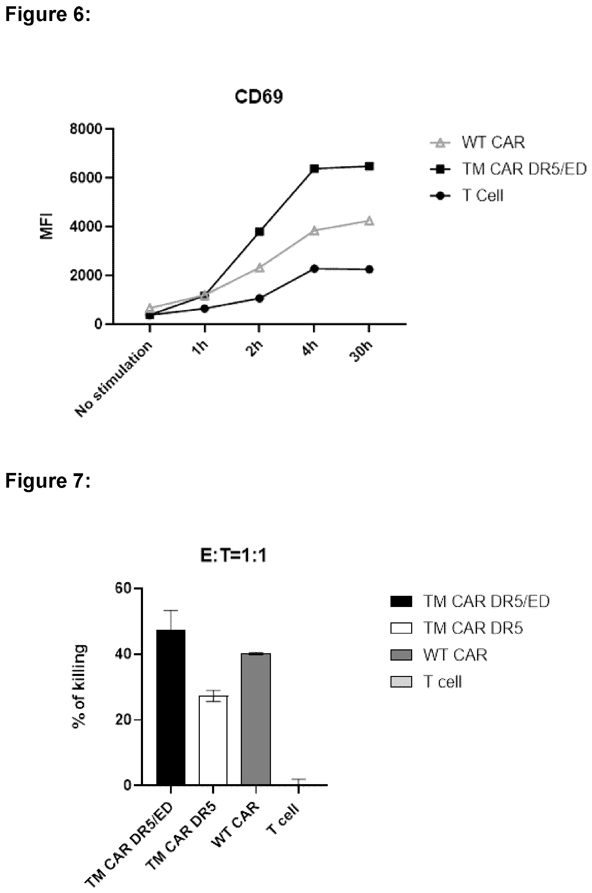

**Figure 6****: Activation assay determination of CD69 expression.**
   Measurements were performed using flow cytometry. As baseline T cells and CAR-T cells were assessed without any stimulation. The effector (CAR-T) and target cells (Raji-Fluc) were cocultured in a ratio of 1:1. Cocultures were measured after 1h, 2h, 4h, and 30h.
**Figure 7****: Killing Assay: CAR constructs with different DR5 transmembrane domain and DR5 hinge and transmembrane domain combinations**
   A scheme of CAR constructs targeting CD19, each incorporating the DR5 transmembrane domain (TM CAR DR5 in white), the transmembrane and hinge domain from DR5 (TM CAR DR5/ED in black), and WT CAR in gray. The effector (CAR-T) and target cells (Raji-Fluc) were cocultured in a ratio of 1:1. Bioluminescence was measured after 24h.

### DEFINITIONS

The term "**cancer immunotherapy**" used herein relates to a treatment that utilizes the body's immune system to combat cancer. This may be achieved through immune cells, antibodies, or immune-modulating agents.

The term "**cell**", used herein, refers to a basic structural unit of a living being; we are talking about eukaryotic cells, more specifically human or animal primary cells or cell lines and/or human t lymphocytes isolated from a donor. In this text, the cancer cells refer to cells that underwent oncogenic transformation and are now kept as genetically identical immortalized clones or cell lines. The term "**cells**" also refers to human or animal primary cells or cell lines. Naturally, the descendants of one cell are not necessarily completely identical to the parents in morphological form and its DNA complement, due to the consequences of natural, random or planned mutations. A "genetically modified host cell" (also "recombinant host cell") is a host cell into which the nucleic acid has been introduced. The eukaryotic genetically modified host cell is formed in such a way that a suitable nucleic acid or recombinant nucleic acid is introduced into the appropriate eukaryotic host cell. The invention hereafter includes host cells and organisms that contain a nucleic acid according to the invention (transient or stable) bearing the operon record according to the invention. Suitable host cells are known in the field and include eukaryotic cells. It is known that proteins can be expressed in cells of a variety of organisms including human, rodent, cattle, pork, poultry, rabbits and the like. Host cells may include cultured cell lines of primary or immortalized cell lines.

The term "**T cell**" used herein refers to T lymphocytes, a subset of white blood cells and a specific population of mononuclear immune cells that are part of the adaptive immune system. Their function is to identify foreign antigens presented by major histocompatibility complex (MHC) molecules via the T cell receptor (TCR). The recognition of antigenic peptides by TCR initiates signaling pathways, resulting in cytokine signaling and the formation of an immune synapse.

The term "**CAR**" used herein refers to a "chimeric antigen receptor", a fusion protein that is either transiently or stably expressed in T cells. CAR is a synthetic receptor composed of an extracellular antigen recognition domain, a hinge domain, a transmembrane domain, and intracellular signaling domains. It is localized on the plasma membrane of T cells, enabling them to identify tumor-specific surface molecules independently of MHC molecules, and is utilized in cancer treatment. Upon recognizing tumor surface antigens, it activates T cells. The specific antigen recognition by CARs leads to T-cell activation, which sequentially kills tumor cells.

The term "**CAR wt**" used herein denotes a second-generation CAR construct composed of a single-chain variable fragment (scFv), a CD8α transmembrane and hinge domain, a 4-1BB costimulatory domain, and a CD3ζ signaling domain. This serves as the standard, unaltered CAR design used for comparison in research studies.

The term "**CAR T cells**" used herein relates to all T cells that bear coding sequence for CAR expression and have therapeutic effect of CAR T cells, in particular anticancer therapeutic effect.

The term "**CAR T cell therapy**" used herein relates to an immunotherapy approach wherein a patient's T cells are genetically modified to express a chimeric antigen receptor (CAR), enabling them to target and kill cells, in particular cancer cells.

The term "**recombinant**" used herein means that a particular nucleic acid (DNA or RNA) results from various methods such as cloning, restriction and/or ligation, or chemical synthesis, leading to a construct with coding or non-coding sequences that differ from natural endogenous nucleic acids in a host system.

The term "**transmembrane domain**" (TMD) used herein describes the hydrophobic region of a CAR construct that spans the lipid bilayer of the T cell membrane, enabling the extracellular domain to detect antigens and the intracellular domain to transmit activation signals. It is typically a simple α-helix, with the task of anchoring the CAR in the membrane and sequestering the oligomerization of the molecule.

The term "**hinge domain**" used herein is the domain that connects the scFv and the transmembrane domain. Hinges are very flexible and extended enabling the scFvs to reach different types of antigens.

The term "**CD8α**" used herein relates to the CD8 (cluster of differentiation 8) costimulatory receptor, from which the hinge and transmembrane domain (TMD) of a wild-type CAR (wt CAR) are derived. CD8 is a transmembrane glycoprotein that functions as a coreceptor to TCR. It is found on cytotoxic T cells and binds to MHC class 1.

The term "**intracellular signaling domain**" used herein pertains to the portion of the CAR construct located within the T cell that conveys signals upon antigen binding. This signaling typically activates pathways that lead to T-cell activation, cytokine production, and cytotoxic responses.

The term "**costimulatory domain**" used herein refers to the intracellular signaling domain in CAR constructs that delivers a secondary signal to enhance T cell activation, persistence, and functionality, fostering a more potent and/or robust response, e.g. anti-tumor immune response in CAR T cell therapy.

The term "**41BB costimulatory domain**" used herein refers to an intracellular signaling domain derived from the 41BB receptor, part of the tumor necrosis factor receptor family. It enhances T cell activation, survival, and memory formation, contributing to sustained anti-tumor activity in CAR T cell therapies.

The term "**CD3ζ activation domain**" used herein refers to an intracellular signaling domain derived from the TCR, specifically, it is derived from the CD3ζ chain of the TCR. It provides the first signal of CAR activation.

The term "**DR5 transmembrane domain**" used herein refers to the TMD that replaces CD8 TMD in this invention it is taken out of the death receptor 5. It is a lipid bilayer spanning α-helix that can for homo dimers and trimers modeling the individual CAR receptors into an effective hexagonal network that promotes clustering and signal amplification.

The term "**DR5 hinge domain**" used herein refers to the ectodomain of the DR5 receptor its job is to connect and hold the scFv and to simultaneously help the TMD with correct oligomerization of the molecules.

The term "**DRS receptor**" used herein refers to the death receptor 5 a receptor belonging to the tumor necrosis factor receptor superfamily its ligand is TRAIL and when bound it triggers oligomerization and activation of the death domains which activate caspase-8.

The term "**protein**" as used herein describes a polymeric chain of amino acids of any length, capable of performing various functions such as localization to specific sites, DNA sequence recognition, catalyzing chemical reactions, transcription regulation, providing structural support, or facilitating biological interactions.

The term "**antibody**" as used herein refers to a protein that can bind to other molecules due to its amino acid sequence and the conformations of its loop regions. These proteins, part of the immunoglobulin family, also include fragments such as variable antibody domains in two chains, single chains, nanobodies, and their binding fragments composed of a single heavy chain.

The term "**scFv**" used herein refers to a "single chain variable fragment," a recombinant antibody fragment comprising the variable regions of both heavy and light chains fused into a single polypeptide chain, used in CAR constructs for antigen binding.

The term "**single chain diabody**" as used herein refers to a recombinant antibody fragment composed of two single-chain variable fragments (scFvs) connected by short linker sequences that prevent intrachain pairing, enabling the formation of functional dimers. Single-chain diabodies may be bispecific, recognizing two different antigens, or monospecific, binding the same antigen with increased valency.

The term "**functional variant**" of a peptide or a peptide fragment, as used herein, refers to a peptide whose amino acid sequence has been modified compared to the original peptide, yet retains the essential biological functions of the original peptide or peptide fragment. Such modifications may include substitutions, deletions, or additions of amino acids that do not significantly impair its functionality. Thus, the functional variant retains at least 20%, at least 30%, at least 40%, at least 50%, at least 60%, at least 70%, at least 80%, at least 90%, or at least 95% of the functionality of the original peptide.

The term "**CD19**" used herein relates to a cell surface protein expressed on B cells and most B cell malignancies, which serves as a target for CAR T cells in the treatment of autoimmune diseases (e.g. systemic Lupus Erythematosus, Myasthenia gravis and Multiple sclerosis) and hematologic cancers like leukemia and lymphoma, as it is specifically expressed on tumor cells in these diseases.

The term **"HER2"** (Erb2) used herein relates to a receptor tyrosine kinase protein overexpressed in several cancer types, such as breast cancer, and is commonly targeted in CAR T cell therapies to eliminate tumors overexpressing this antigen, which plays a role in promoting tumor cell proliferation and survival.

The term **"BCMA"** used herein relates to B-cell maturation antigen, a cell surface protein expressed on plasma cells and some B cell-derived cancers like multiple myeloma, which serves as a target for CAR T cell therapies aiming to selectively kill malignant plasma cells.

The term **"target cell"** used herein relates to a cell, such as a tumor cell, that is specifically recognized and attacked by effector immune cells, such as CAR T cells, in therapeutic applications.

The term **"effector cell"** used herein relates to immune cells, such as CAR T cells, that are capable of directly attacking and killing target cells, such as cancer cells, by recognizing specific antigens on the tumor surface.

The term **"killing"** used herein relates to the process by which immune cells, including CAR T cells, induce apoptosis or direct cytotoxicity in tumor cells through mechanisms such as perforin/granzyme release.

The term **"persistence"** used herein relates to the ability of T cells to survive, proliferate, and maintain their functional activity over extended periods, which is critical for the long-term efficacy of CAR T cell therapy in eradicating tumors.

The term **"exhaustion"** used herein relates to the dysfunctional state of T cells after prolonged activation, characterized by a reduced ability to produce cytokines and kill target cells, commonly occurring in the context of chronic infections or cancer.

The term **"antigen recognition"** used herein relates to the binding of a specific antigen by a receptor, such as a CAR or TCR, on immune cells, which initiates signaling events that trigger immune responses such as T cell activation and tumor cell elimination.

The term **"identity"** refers to the sequence identity of nucleic acid or amino acid sequences. Identity determination can be done, for example, by a sequence alignment based on well-established and commonly used BLAST algorithms. Such an alignment is based on aligning similar nucleotide or amino acid sequences stretches with each other. Alignments, particularly multiple sequence comparisons, are typically done using computer programs such as the Clustal series. Such comparisons allow determination of the similarity of the compared sequences. Said similarity is typically expressed in percent identity, i.e. the portion of identical nucleotides/amino acids at the same or corresponding (in an alignment) sequence positions relative to the total number of the aligned nucleotides/amino acids. For example, if in an alignment 90 amino acids of a 100 amino acid long query sequence are identical to the amino acids in corresponding positions of a template sequence, the sequence identity is 90%. The broader term "homology" additionally considers conserved amino acid substitutions, i.e. amino acids that are similar regarding their chemical properties, since those typically have similar chemical properties in a protein. Unless otherwise specified, sequence identity and homology are calculated over the total length of the aligned sequences and are expressed as a percentages.

The insertion of vectors into host cells is achieved through conventional scientific methods, including transformation or transfection techniques such as chemical induction, electroporation, microinjection, DNA lipofection, cellular sonication, gene bombardment, viral DNA transfer, and others. DNA entry may be transient or stable. Transient insertion involves DNA delivery via a vector that does not integrate into the cell's genome. Stable insertion occurs when DNA is incorporated into the host genome, allowing replication and transmission to progenitor cells. Screening for stable insertion can involve detecting markers, with marker DNA sequences conferring resistance to antibiotics or chemicals. These sequences may be included on the DNA vector or on a separate vector.

The implementation examples that are described in more detail are designed to best describe the invention. These descriptions have no intention of limiting the scope of the invention and its applicability but are merely intended to provide a better understanding of the invention and its use.

### DETAILED DESCRIPTION OF THE INVENTION

This innovation refers to a modified second-generation chimeric antigen receptor (CAR) that incorporates the transmembrane and hinge regions of Death Receptor 5 (DR5-based CAR), replacing the CD8 transmembrane and hinge regions. The original second-generation CAR comprises a scFv, a CD8α hinge, and a transmembrane region, which have been replaced with the DR5 hinge and transmembrane domain in this modification. On the cytosolic side, the costimulatory 4-1BB domain and the CD3ζ domain are positioned adjacent to the transmembrane domain. The hinge region is located on the extracellular side and is connected to the scFv of choice, while the transmembrane domain spans the lipid bilayer.

This modification enhances the tumor-killing capacity of the CAR constructs, as demonstrated in both in vivo and in vitro models targeting CD19. The DR5-based CAR exhibited enhanced anti-tumor activity and stronger activation, as evidenced by increased expression of the activation marker CD69 (Cibrián and Sánchez-Madrid 2017), compared to conventional CAR with CD8 transmembrane and hinge regions. Furthermore, in vitro testing with CARs targeting BCMA confirmed the enhanced efficacy of the DR5-based CAR, demonstrating its versatility.

The invention utilizes the higher-order oligomer forming transmembrane and the hinge region of the DR5 receptor which is well characterized (Pan et al. 2019). The sequence (SEQ ID NO: 63) of this receptor is available in UniProt (Accession Number: O14763). Unlike the conventional CD8 transmembrane domain, which dimerizes, the DR5 domain enables the formation of larger CAR signaling clusters.

DR5 receptor is known to form a network of dimer-trimer interactions, all facilitated by the trimer-dimer interfaces in the receptor transmembrane region. More specifically, for dimer formation glycine residues (G213, G217) which form a GXXXG motif (Mueller, Subramaniam, and Senes 2014; Russ and Engelman 2000) that form dimers with glycines of the opposite chain, respectively, indicating close glycine packing. In contrast, T219 and A222 are responsible for the trimerization of the same transmembrane domain. All of these elements are present in our new domain. It is hypothesized that in natural DR5 receptors, the oligomerization takes place upon ligand binding. The binding of the TRAIL ligand enables overcoming the inhibitory restraint that the ectodomain (ECD) (56-210 amino acids of SEQ ID NO: 63) places on the transmembrane region by altering the pre-ligand conformation (Pan et al. 2019). Since our end CAR construct does not have an ECD, oligomerization occurs spontaneously. The introduced TM (177-211 amino acids of SEQ ID NO: 63 and ECD regions improve the lateral movement within the plasma membrane, leading to efficient signaling. The transmembrane domain from the DR5 receptor also enables the CAR a superior location within microdomains such as lipid rafts, this might improve the assembly in the T cell receptor signaling complex, leading to an improved immunological synapse formation.

The use of a native hexamer-forming domain in this context has not yet been explored and was unknown if it could function as it might trigger constitutive activation of CARs, which was not desired. Therefore, it was surprisingly observed that the subdomains of DR5, which has not yet been investigated in the context of CARs did not have constitutive activity and even triggered better activation in comparison to the used CD8 transmembrane and hinge domains. Its transmembrane sequence bears little similarity to the domain of TNFRSF16 and TNFRSF19, which has been investigated before.

The introduction of a dimer-trimer forming oligomerization domain therefore represents a surprising advancement in CAR functionality by improving cell activation and tumor clearance.

Selection of native TMDs provides distinct advantages over synthetic alternatives as described by Elazar et al. (2022). In particular, the use of a natural transmembrane domain may reduce the risk of immunogenicity by retaining greater similarity to endogenous human proteins, thus minimizing the likelihood of eliciting an immune response. This property is particularly advantageous in therapeutic applications where long-term persistence and low immunogenicity of the engineered cells are critical for efficacy and safety. The selection of a natural transmembrane domain therefore represents a strategic design decision to improve the clinical translation and patient tolerability.

Thus, in the first aspect a chimeric antigen receptor (CAR) is provided that comprises hinge domain and/or a transmembrane domain derived from a mammalian DR5 receptor, more preferably, which is derived from a human DR5 receptor domain.

The inventive CAR preferably comprises the components:
(i) an extracellular antigen binding domain that targets the selected antigen or a selected cell type expressing the target antigen,
(ii) a hinge domain derived from the ectodomain of a mammalian DR5 receptor,
(iii) a transmembrane domain derived from a mammalian DR5 receptor,
(iv) a co-stimulatory domain, and
(v) an activating domain,
and wherein components (i), to (v) are preferably comprised in N- to C-terminal order. A N- to C-terminal order of (i) to (v) is in particular preferred.

The chimeric antigen receptor comprises preferably an antigen binding domain (i) comprising a functional binding fragment of a natural binding protein, in particular a receptor or antibody, preferably a single-chain variable fragment (scFv) of an antibody; or a short synthetic protein that binds to the selected antigen, such as machine learning-based designed binder proteins (aka 'mini binder') or DARPINs. The antigen binding domain (i) is preferably of mammalian and in particular human origin.

The chimeric antigen receptor comprises preferably a hinge (ii) and/or transmembrane domain (iii) of a mammalian death receptor 5 (DR5), preferably human DR5, or functional fragments thereof having at least 75% identical residues to human DR5.

In specific embodiments, components (ii) and (iii) are joined either directly or via a short flexible peptide linker.

The chimeric antigen receptor comprises preferably a co-stimulatory domain or functional variant of component (iv) preferably having at least 75% identical residues to the co-stimulatory domain of human 4-1 BB or CD28, in particular to the sequence of SEQ ID NO: 39 (4-1BB), and/or 64 (CD28).

The chimeric antigen receptor comprises preferably an activating domain or functional variant of (v) preferably having at least 75% identical residues to the human sequence of SEQ ID NO: 40 (CD3ζ).

In specific embodiments, components (iv) and (v) are joined either directly or via a short flexible peptide linker.

The chimeric antigen receptor comprises preferably an activating domain (v) comprising a CD3ζ intracellular domain or another domain that comprises two or more ITAM motifs, which are phosphorylated upon recognition of cells with target antigens.

In specific embodiments, components (iv) and (v) may also be arranged in reverse order, with component (vi) positioned C-terminally to component (v).

The chimeric antigen receptor comprises preferably an antigen binding domain (i) that specifically binds an antigen on the surface of a pathologic cell, preferably a tumor associated antigen, optionally selected from CD19, BCMA, or ErbB2 (HER2).

The chimeric antigen receptor comprises preferably a DR5 receptor derived hinge domain (ii) comprising the amino acid amino acid sequence of SEQ ID NO: 1 or a functional variant thereof having at least 75% sequence identity.

The chimeric antigen receptor comprises preferably a DR5 receptor derived transmembrane domain (iii) comprising the amino acid amino acid sequence of SEQ ID NO: 3 or a functional variant thereof having at least 75% sequence identity.

The chimeric antigen receptor comprises preferably the amino acid sequences of SEQ ID NOS: 5-10 or functional variants thereof having at least 75% sequence identity, and optionally variants thereof lacking the Myc tag and/or the leader peptide.

A nucleic acid, or a plurality of nucleic acids are provided encoding the inventive chimeric antigen receptor (CAR) fusion proteins claimed.

The nucleic acid encoding the CAR preferably comprises
(A) a sequence encoding SEQ ID NO: 1 and/or SEQ ID NO: 3 or functional variants thereof having at least 75% sequence identity
(B) a sequence encoding any one of SEQ ID NOs: 5-10, or
   functional variants thereof having at least 75% sequence identity

A recombinant expression vector comprising the inventive encoding nucleic acids is provided, wherein the expression vector is optionally a viral vector, a transposon or a mRNA, each optionally configured for delivery to target cells.

An isolated mammalian cell comprising the inventive expression vector or being engineered to express the inventive encoding nucleic acid is provided. The corresponding host cell is preferably selected from the group consisting of an αβ T cell, a γδ T cell, a natural killer (NK) cell, a natural killer T (NKT) cell, a cytotoxic T lymphocyte (CTL), a regulatory T cell, a macrophage or combinations thereof.

A pharmaceutical composition comprising the CAR, the nucleic acid or recombinant vector, or the isolated mammalian cell or population thereof, all as described above, and a pharmaceutically acceptable carrier and/or excipient is provided.

A method of treating, preventing, or ameliorating a condition or disorder associated with the expression of an antigen, preferably a tumor antigen, in a subject is provided. The method comprises administering to the subject an effective amount of the CAR, and/or the nucleic acid or recombinant vector, and/or the isolated mammalian cell or population thereof, and/or the pharmaceutical composition, all provided as described above.

The CAR, and/or the nucleic acid or recombinant vector, and/or the isolated mammalian cell or population thereof, and/or the pharmaceutical composition, all as described above are provided for use in treating, preventing and/or ameliorating a condition or disorder associated with the expression of an antigen, preferably a tumor antigen.

In a preferred embodiment, the herein disclosed improved CAR constructs comprise both the hinge and transmembrane domains derived from the human DR5 receptor (SEQ ID NO: 63). Without being bound by theory, it is believed that the coordinated interplay of structural features within these domains facilitates receptor oligomerization. In particular, the transmembrane domain exhibits an inherent capacity to mediate both dimer and trimer formation, thereby promoting receptor clustering and enhancing CAR activation efficiency.

Receptor oligomerization is in particular facilitated by the DR5 transmembrane domain (SEQ ID NO: 3, which enables tight dimer formation via interhelical glycine packing at positions G213 and G217 within the GXXXG motif and promotes stabilization of associated trimeric structures via residues T219 and A222.

For the purpose of the invention, a preferred CAR comprises the native DR5 transmembrane domain of SEQ ID NO: 3, or a functional variant thereof, that differs from the native DR5 transmembrane sequence by amino acid substitutions, insertions, or deletions of one or more amino acids, provided that it retains at least the dimerization-promoting glycines (G213 and G217) of the GXXXG motif and/or the trimerization-facilitating residues T219 and A222. Preferably, such functional variants have an amino acid sequence identity or homology, preferably identity, of at least 75%, at least 80%, 81%, 82%, 83%, 84%, 85%, 86%, 87%, 88%, 89%, 90%, 91%, 92%, 93%, 94%, 95%, 96%, 97%, 98%, or 99% with SEQ ID NO: 3.

It is further understood that the DR5 hinge (SEQ ID NO: 1), or a functional variant thereof, may be used either in combination with the DR5 transmembrane domain or with other transmembrane domains suitable for CAR design. Preferably, the hinge is used in combination with the human DR5 transmembrane domain. Functional hinge variants preferably have an amino acid sequence identity or homology, preferably identity, of at least 75%, at least 80%, 81%, 82%, 83%, 84%, 85%, 86%, 87%, 88%, 89%, 90%, 91%, 92%, 93%, 94%, 95%, 96%, 97%, 98%, or 99% with SEQ ID NO: 1.

In a preferred embodiment, the C-terminal end of the hinge domain (preferably DR5 hinge) is joined to the N-terminal end of the transmembrane domain (preferably DR5 transmembrane domain), either directly or via a short peptide linker. Suitable linkers are known in the art and are preferably selected from flexible glycine- and/or serine-rich linkers having a length of 1-20 amino acids.

In a particularly preferred embodiment, the hinge and transmembrane domains of the CAR are provided as a contiguous amino acid sequence consisting of the DR5 hinge domain and the DR5 transmembrane domain in their native sequence and orientation, corresponding to SEQ ID NO: 1 conjugated to SEQ ID NO: 3.

In the context of the herein disclosed CAR of the invention, the extracellular antigen-binding domain (i) refers to a functional "antigen-binding fragment" of an antibody, which is defined as a portion of an intact antibody that retains the ability to bind the same antigen as the intact antibody. Examples of antibody fragments include, but are not limited to, Fv, Fab, Fab', Fab'-SH, F(ab')₂; diabodies; linear antibodies; nanobodies (e.g., VHH); single-chain antibody molecules (e.g., scFv or single-chain diabodies); and multi-specific antibodies formed from antibody fragments.

In the context of the herein disclosed CAR of the invention, the extracellular antigen-binding domain (i) may also be a natural binding protein or functional fragment thereof, such as a receptor or a functional variant of the binding domain of a receptor. Alternatively, it may be a short protein with specific binding activity, such as a Designed Ankyrin Repeat Protein (DARPIN), which consists of engineered ankyrin repeat motifs that form a stable, high-affinity binding scaffold, or a designed protein binder domain (aka minibinder).

As used herein, a 'designed protein binder domain' refers to a computationally designed polypeptide that specifically binds to a target molecule, such as an antigen, through a structurally optimized binding interface. Unlike naturally occurring binding proteins, designed minibinders are generated *de novo* using structure-based computational design methods to achieve high binding affinity, specificity, and stability. These polypeptides typically adopt a well-defined three-dimensional fold, such as a helical bundle or β-sheet architecture, and may be engineered to resist denaturation and proteolytic degradation. Methods for providing minibinders are known in the art, e.g. WO2016005969A1.

In the context of the CAR disclosed herein, antibody-binding fragments are preferably designed such that all sequences required for antigen binding, for example, the variable light chain (VL) and the variable heavy chain (VH) of an antibody-binding site, are arranged on a single polypeptide chain (single-chain). Accordingly, the antigen-binding domain (i) is preferably configured as an scFv, single-chain diabody, or nanobody.

In preferred embodiments, the antigen-binding domain (i) is an scFv fragment.

Although embodiments comprising both the hinge and the transmembrane domain of a mammalian - preferably human - DR5 receptor are preferred in the context of the present invention, the inventive CAR construct may also comprise a DR5 transmembrane domain in combination with a non-DR5 hinge domain, or a DR5 hinge domain in combination with a non-DR5 transmembrane domain.

In the context of such CAR constructs comprising either a non-DR5 hinge domain and a DR5 transmembrane domain, or a DR5 hinge domain and a non-DR5 transmembrane domain, the respective non-DR5 domain is not particularly limited and may be selected from a broad range of known domains derived from mammalian transmembrane proteins, in particular from membrane-bound receptors, and more preferably from membrane-anchored proteins that are naturally expressed on T cells.

According to the invention, such non-DR5 hinge and/or transmembrane domains are selected from the group comprising the transmembrane regions of the alpha, beta, or zeta chain of the T cell receptor (TCR), CD28, CD3 epsilon, CD45, CD4, CD5, CD8, CD9, CD16, CD22, CD33, CD37, CD64, CD80, CD86, CD134, CD137, and CD154. Particularly preferred are IgG- and CD28α-derived domains.

Alternatively, the transmembrane domain can be synthetic, in which case it will comprise predominantly hydrophobic residues such as leucine and valine. In several embodiments, a triplet of phenylalanine, tryptophan and valine will be found at each end of a synthetic transmembrane domain.

The hinge (ii) and transmembrane domain (iii) link the extracellular antigen binding domain (i) to the intracellular co-stimulatory and/or signaling moiety of the CAR. On its N-terminal end the hinge moiety may be either joined directly to the C-terminal end of the antigen binding fragment (i) or joined via a short amino acid sequence having a length of e.g.1-50 amino acids.

To facilitate optimal binding of the antigen-binding domain (i) to sterically complex targets, for example, those on the surface of cancer cells, the hinge domain (ii), preferably DR5 hinge domain, is configured as a flexible spacer or comprises an additional flexible spacer. This spacer is preferably a naturally occurring hinge as described above or synthetic peptide with a length of up to 300 amino acids, for example, 10 to 200 amino acids, preferably 15 to 100 amino acids, and more preferably 25 to 50 amino acids.

Without being bound by theory, it is believed that suitable naturally occurring spacers are flexible protein domains that allow optimal orientation of the antigen binding domain, for example fragments of the Ch2-CH3 domains of IgG antibodies.

Other suitable naturally occurring spacers include hinge fragments of membrane-anchored T-cell proteins, such as CD4, CD8, CD28, or CD3-epsilon, or functional variants thereof.

In specific embodiments, component (ii) and (iii) is configured to comprise a partial ectodomain and transmembrane domain of a mammalian DR5 receptor (177-236 amino acid residues of SEQ ID NO: 63 or a functional fragment thereof spanning at least 90%, preferably at least 80%, 70%, 60%, 50%, 40%, 30%, 20%, or 10% of said ectodomain, more preferably of the human DR5 receptor.

In specific embodiments, component (iii) is configured without a spacer.

For optimal binding of (i) to the target antigen, component (iii) is preferably SEQ ID NO: 3 with an additional spacer (ii).

Although the DR5 transmembrane domain in preferred CAR configurations contributes to receptor oligomerization, its primary role - from a structural perspective - is to anchor the receptor in the cell membrane. It ensures stability and proper orientation of the extracellular and intracellular domains and contributes to signal transduction by facilitating the spatial assembly of the CAR complex and associated signaling molecules.

In embodiments with a hinge moiety, the N-terminal end of the transmembrane moiety is joined to the C-terminal of the hinge moiety. In configurations without a hinge moiety, the transmembrane moiety might be bound directly to the C-terminal end of antigen-binding component (i). At its C-terminal end on the cytoplasmic side of the plasma membrane, the transmembrane domain is preferably joined to the intracellular co-stimulatory domain (iv), or, in certain configurations, to the T-cell activating domain (v).

In preferred embodiments, a CARs disclosed herein is designed such that the C-terminal end of component (iii) is bound directly or by utilization of a short peptide linker to the N- terminal end of a co-stimulatory domain (iv) or to a CD3ζ signaling domain (v).

Configurations wherein component (iii) is bound directly to the N- terminal end of a co-stimulatory domain are preferred.

Efficient receptor oligomerization enhances the activity of the cytosolically located co-stimulatory and CD3ζ-like signaling domains of second-generation CARs, which together control key aspects of CAR T-cell functionality, including activation, proliferation, and persistence. The therapeutic efficacy of these CAR T cells is therefore largely determined by the finely tuned interplay of signaling events driven by receptor oligomerization, which helps ensure sufficient signal initiation even under limiting conditions, such as when target cells present low levels of antigen.

In second-generation CARs, co-stimulatory domains such as CD28 and 4-1BB (CD137) are preferably used and have proven effective in enhancing T-cell function. CD28 promotes rapid and robust T-cell activation, whereas 4-1BB supports sustained T-cell persistence and resistance to exhaustion.

Furthermore, expanding the repertoire of co-stimulatory domains has shown to offer additional therapeutic opportunities. OX40 (CD134) enhances the long-term survival and function of T cells, making it relevant for applications focused on sustained effects. ICOS promotes a balanced T-cell response and plays a critical role in the differentiation of helper T cells. HVEM amplifies cytotoxic activity and T-cell persistence, potentially increasing effectiveness in tumor models. CD27 supports T-cell activation and proliferation, particularly in therapeutic approaches aimed at eliciting rapid and robust immune responses. GITR fosters both proliferation and functionality of T cells, especially in combination with other signaling pathways, offering a versatile option for CAR T-cell optimization.

The targeted selection and integration of these co-stimulatory domains promises precise adaptation of CAR T cells to specific clinical requirements, thus contributing to the enhancement of the therapeutic efficacy and safety of related treatments.

In the context of the present invention, the utilized co-stimulatory domain (iv) is not limited to the corresponding domains of the aforementioned CD28, 4-1BB, OX40, ICOS, HVEM, CD27, and GITR, or their functional variants. Rather, a skilled person may identify additional advantageous co-stimulatory domains, for example, based on their effects on T-cell proliferation and/or persistence.

In preferred embodiments, the co-stimulatory domain of component (iv) is selected from the co-stimulatory domains of the proteins CD28 or 4-1BB, preferably of human origin in accordance with SEQ ID NO:64 and 39.

The disclosed CARs often include primary cytoplasmic signaling sequences that act in a stimulatory manner, which may contain signaling motifs that are known as immunoreceptor tyrosine-based activation motifs or ITAMs. Examples of ITAM containing primary cytoplasmic signaling sequences that can be included in a disclosed CAR include those from CD3ζ, FcRγ, CD3 γ, CD3δ, CD3ε, CD79(a or b), and CD66d proteins.

In preferred embodiments, the cytoplasmic activating domain of the CAR may comprise an intracellular T cell signaling domain from CD3ζ. It is preferred that the activating CD3ζ domain comprises three ITAM motifs, corresponding to its naturally occurring form. Depending on the application, the CD3ζ domain may alternatively comprise only one or two ITAM motifs to elicit a less intense T-cell activation, for example.

It is preferred that the activating domain (v) of the CAR comprises the amino acid sequence of SEQ ID NO: 40 or a functional variant thereof.

The disclosed CAR is preferably a single-chain polypeptide comprising, in an N-terminal to C-terminal order, the following components: (i) an extracellular antigen-binding domain, (ii) a hinge domain, preferably derived from human DR5, (iii) a transmembrane domain preferably derived from human DR5, (iv) a co-stimulatory domain, and (v) an activating domain. To ensure proper processing and translocation to the cell membrane, preferred embodiments provide for a leader peptide (signal peptide) at the N-terminal end of this polypeptide. This leader peptide may optionally comprise a short sequence, preferably 1-50 amino acids in length.The leader sequence is not specifically limited, as a variety of known leader sequences from transmembrane proteins are suitable.

In preferred embodiments, the CD8 leader sequence according to SEQ ID NO: 42 or a functional variant thereof, is selected.

In another embodiment, the CAR is not configured as a single-chain polypeptide; rather, its functionality is distributed across multiple, preferably two, polypeptide chains, for example in a split CAR configuration. Split CARs are preferably composed of two separate protein components that become functional only upon reassembly within the target cell. This modular design enables controlled CAR activation and often allows for the reduction of off-target activity.

In the case of a non-single-chain configuration, the CAR may be encoded by a single nucleic acid comprising multiple coding sequences separated, for example, by IRES elements or ribosome-skipping units, or alternatively by a plurality of nucleic acids, e.g., a mixture of at least two separate RNA molecules encoding distinct CAR subunits.

The effectiveness of CAR T-cells lies in their ability to specifically recognize target cells and eliminate them by employing one or more mechanisms inherent to or triggered by the CAR T-cell. This includes direct cytotoxicity (e.g., granzyme-induced apoptosis), cytokine release, and the recruitment of additional immune cells, making CAR-T therapies a highly effective treatment for proliferative diseases, particularly hematological malignancies.

A critical aspect of CAR T-cell-mediated elimination of target cells lies in their highly specific antigen recognition, which in turn relies on the incorporated antibody-derived antigen-binding fragments (typically single-chain polypeptides comprising the variable domains, preferably scFvs) within the CAR structure. These scFvs are designed to bind with high specificity to predefined target antigens expressed on the surface of a malignant cell or a specific cell type. This antigen-specific recognition not only triggers T-cell activation but also minimizes off-target effects, a crucial factor for the safety and efficacy of CAR-T therapies.

The modular structure of CARs enables the simple integration of antigen-binding fragments for a broad repertoire of already validated target antigens. This adaptability leverages extensive research in the field of antibodies, enabling a skilled person to tailor CAR-T cells for diverse therapeutic applications.

In the context of the present invention, the specificity of the antigen-binding domain (i) is not restricted. Rather, a skilled person can select from a variety of antigen-binding domains when designing an appropriate CAR, preferably focusing on antibody fragments that have been therapeutically validated. Attractive antigens within the scope of this invention include tumor-associated antigens (TAAs) as well as antigens expressed on autoreactive cells, such as autoreactive B cells. A non-limiting list of TAA that are predominantly associated with solid tumors comprises Erb2 (HER2/neu), EGFR, EGFRvIII, MUC1, mesothelin, CEA, PSMA, GD2, GPC3, NY-ESO-1, WT1, AFP, CA125, TAG-72, B7-H3, PD-L1, Trop2, Trop3, or CD95. Similarly, the invention encompasses TAAs typically associated with hematological malignancies, such as CD19, CD20, CD22, CD30, CD33, CD123, BCMA, FLT3, CLL-1, and SLAMF7.

It is preferred that the extracellular antigen-binding domain (i) is selected from antibodies specifically binding to CD19, ErbB2, or BCMA.

In preferred embodiments, the extracellular antigen-binding domain (i) is selected from single-chain antibody fragments, especially scFvs, specifically binding to CD19, ErbB2, or BCMA, optionally having the amino acid sequence of SEQ ID NO: 53, 62 or 66, or a sequence inherently disclosed as binding domain of the CARs of SEQ ID NOs: 5-10, or is defined by the VH/VL or CDR sequences of SEQ ID NOs: 45-52, or 54-61, or a functional variant thereof.

The chimeric antigen receptor (CAR) of the invention preferably comprises in an N- to C-terminal arrangement,
an antigen-binding scFv fragment,
linked to a hinge region of SEQ ID NO: 1,
linked to a transmembrane domain of SEQ ID NO: 3,
linked to an intracellular co-stimulatory moiety preferably of SEQ ID NO: 39, or 64,
linked to an intracellular activating moiety of SEQ ID NO: 40
wherein the scFv is selected from SEQ ID NO: 53, 62, or 66,
   and
wherein the CAR optionally comprises an N-terminal located leader peptide of SEQ ID NO: 42, and/or a Myc-tag of SEQ ID NO: 41,
or functional variants thereof having at least 75%, at least 80%, 81%, 82%, 83%, 84%, 85%, 86%, 87%, 88%, 89%, 90%, 90%, 91%, 92%, 93%, 94%, 95%, 96%, 97%, 98%, 99%, sequence identity or homology, preferably identity, to said CAR.

The chimeric antigen receptor (CAR) of the invention comprises preferably the amino acid sequences of any one of SEQ ID NOs 5-10 or functional variants thereof, optionally incl. variants lacking the Myc tag, comprising a sequence:
**(A)** that is at least 75% at least 80%, 81%, 82%, 83%, 84%, 85%, 86%, 87%, 88%, 89%, 90%, 90%, 91%, 92%, 93%, 94%, 95%, 96%, 97%, 98%, 99% identical to any one of SEQ ID NOs 5-10, or
**(B)** that is at least 75% at least 80%, 81%, 82%, 83%, 84%, 85%, 86%, 87%, 88%, 89%, 90%, 90%, 91%, 92%, 93%, 94%, 95%, 96%, 97%, 98%, 99% identical to the comprised hinge and/or transmembrane domains of any one of SEQ ID NOs 1 and 3.

In a further aspect, the invention relates to nucleic acids encoding the herein disclosed CAR fusion proteins, wherein the nucleic acids are not limited and include nucleic acids in linear or circular form, for example, single-stranded RNA such as mRNA, as well as single- or double-stranded DNA.

Preferred is a nucleic acid encoding a CAR fusion protein as disclosed herein, wherein the nucleic acid comprises:
**(A)** a nucleotide sequence encoding any one of SEQ ID NOs:5-10 or functional variants thereof, or nucleic acids having at least 75% least 80%, 81%, 82%, 83%, 84%, 85%, 86%, 87%, 88%, 89%, 90%, 91%, 92%, 93%, 94%, 95%, 96%, 97%, 98%, or 99% nucleotide sequence identity thereto, or
**(B)** a nucleotide sequence encoding the comprised hinge and/or transmembrane domains of any one of SEQ ID NOs 1 and 3, or a functional variant thereof having at least 75% least 80%, 81%, 82%, 83%, 84%, 85%, 86%, 87%, 88%, 89%, 90%, 91%, 92%, 93%, 94%, 95%, 96%, 97%, 98%, or 99% nucleotide sequence identity thereto,
**(C)** a nucleotide sequence of any one of SEQ ID NOs: 2, 4, and 11-16 or functional variants thereof, having at least 75% least 80%, 81%, 82%, 83%, 84%, 85%, 86%, 87%, 88%, 89%, 90%, 91%, 92%, 93%, 94%, 95%, 96%, 97%, 98%, or 99% nucleotide sequence identity thereto.

To exert a therapeutic effect, the CARs disclosed usually have to be integrated into the cell membrane of a cytotoxic cell in a spatially correct manner. This is preferably achieved through the expression of the encoding nucleic acids in the respective cytotoxic cells.

The expression of nucleic acids encoding the herein disclosed CAR can be achieved by operably linking the DNA encoding the CAR to a promoter (either constitutive or inducible), optionally through integration of the nucleic acid into an expression cassette e.g. of a vector or a plasmid. The promoter can be any promoter of interest, including viral promoters. Optionally, an enhancer might be included in the construct. The cassettes can be suitable for replication and integration in either prokaryotes or eukaryotes.

In a further aspect, the invention relates to a recombinant expression vector comprising a nucleic acid encoding the herein disclosed CAR.

For the expression of the CAR in a cytotoxic host cell, preferably a T cell or NK cell, the encoding nucleic acid must be either permanently integrated into the genome or transiently introduced into the cell. Any suitable coding nucleic acid may be introduced into the cell, for example, via electroporation. Particularly suitable forms include plasmid-based DNA, RNA, or mRNA constructs. Preferred embodiments encompass vectors and plasmids with integrated expression cassettes, as well as viral vectors.

To facilitate cellular uptake, it is preferred to formulate nucleic acids, in particular RNA encoding a CAR as disclosed herein, in a stable form, for example by encapsulation in lipid nanoparticles (LNPs) and/or complexation with cationic lipids, which protect against degradation and enhance transfection. In preferred embodiments, stability may further be increased by incorporating non-natural or chemically modified nucleotides.

When using viral vectors, the selection can take into account the specific requirements of the system. Lentiviral vectors are capable of stable integration into the host genome and support long-term expression, making them suitable for both dividing and non-dividing cells. Retroviral vectors also enable stable expression but are primarily effective in dividing cells. Adenoviral vectors facilitate transient but highly efficient gene expression and are characterized by their large packaging capacity, whereas Sendai viruses offer transient expression without genomic integration and high transduction efficiency in primary cells. Adeno-associated viruses (AAV) are notable for their low immunogenicity and their ability to support both transient and stable expression, though they are limited by their smaller packaging capacity.

For stable expression, lentiviral or retroviral vectors are preferred. For transient expression, adenoviral vectors or Sendai viruses are particularly suitable. For specific requirements, such as low immunogenicity, adeno-associated viruses (AAV) are advantageous.

In the context of the invention, lentiviral vector systems are in particular preferred.

In a further aspect the invention relates to an isolated mammalian cell comprising a CAR as disclosed herein, a vector for the expression of said CAR, or a mammalian cell that has been engineered or genetically modified to express the CAR-encoding nucleic acids disclosed herein. These cells are designed to express the CAR in a targeted and efficient manner, ensuring their suitability for therapeutic applications.

The mammalian cell provided according to the invention is preferably selected from the group consisting of αβ T cells, γδ T cells, natural killer (NK) cells, natural killer T (NKT) cells, cytotoxic T lymphocytes (CTLs), regulatory T cells, macrophages, or combinations thereof. Of course, also any population of such cells are comprised.

Among these, T cells are the best-characterized cell type in the development of therapeutic CARs, owing to their well-established role in adaptive immunity and their ability to mediate highly specific cytotoxic responses against target cells.

NK cells, in contrast, are particularly advantageous due to their excellent safety profile. Unlike T cells, NK cells are less likely to induce severe side effects such as cytokine release syndrome, making them especially suitable for allogeneic therapies. NKT cells combine features of T cells and NK cells, providing hybrid functionality that includes both cytotoxicity and immune modulation.

Regulatory T cells are valuable for suppressing autoimmune reactions and maintaining immune tolerance, while macrophages hold promise in targeting solid tumors through phagocytosis and modulation of the tumor microenvironment.

The diversity of suitable cytotoxic host cell types underscores the broad therapeutic applicability of the herein disclosed CARs.

In a further aspect the invention relates to pharmaceutical composition comprising the herein disclosed CAR, the herein disclosed nucleic acids or recombinant vectors, or the here disclosed isolated mammalian cell or population thereof, and a pharmaceutically acceptable carrier and/or excipient.

Examples of suitable carrier and excipients for formulating compositions comprising the herein disclosed CAR, the herein disclosed nucleic acids or recombinant vectors, or the herein disclosed isolated mammalian cell or population thereof include saline and aqueous buffer solutions and are well known in the art.

Typically, the pharmaceutical composition is adapted for parenteral administration, e.g., for intravenous infusion or injection, which is highly preferred for compositions containing cells. In further embodiments, the pharmaceutical composition is adapted for local administration, such as intratumoral injection, or for regional perfusion to target specific tissues or organs.

The determination of dosage and frequency of administration lies within the professional judgment of the treating physician and may vary depending on the patient's condition, disease stage, and individual response. Typically, the pharmaceutical composition is administered as a single infusion of a therapeutically effective dose, but in some cases, additional doses may be required to achieve or maintain the desired therapeutic effect. For example, the composition may be administered in one or more treatment cycles, with intervals of several weeks to months between infusions, depending on the therapeutic response and tolerability. Re-administration may be necessary in cases of disease progression or suboptimal initial response.

### MEDICAL APPLICATIONS

According to a further aspect of the invention the herein disclosed subject matter, in particular the CAR, the nucleic acid encoding the CAR, the cells comprising the CAR as well as disclosed pharmaceutical compositions is used in medicine, including human and veterinary medicine, particularly in human medicine.

Due to the modular design of the CAR, the therapeutic spectrum of said CAR depends solely on the selected external antigen-binding domain. The skilled artisan will select this domain as needed from the vast array of already characterized antigen-binding domains known in the art. Accordingly, subject matter disclosed herein is particularly suitable for therapies aimed at the elimination or reduction of a specific, typically pathological cell population. For example, they are suitable for the treatment of proliferative diseases, both solid and hematological in nature, provided these are characterized by distinctive surface antigens (TAA).

Relevant diseases are referred to in the context of the invention as diseases "associated with the expression" of a specific antigen. This term encompasses overexpression, decreased/inhibited expression, or even unchanged expression of a target antigen compared to a non-pathological condition, depending on the target and/or the disease to be treated.

"Suitable for therapy" within the meaning of the present invention refers to the use of the entities for the treatment, amelioration, and prevention of the respective disease.

The subject matter of the invention, in particular, provides promising treatment options for individuals who have relapsed or become refractory after prior treatment with one or more medications - often considered the standard of care - including those targeting the same antigen.

### Erb2 (HER2/neu) related disorders

Within the meaning of the present invention, the subject matter described herein with an Erb2-binding antibody domain is suitable for therapeutic application in proliferative or malignant disorders associated with Erb2 expression.

In particular solid tumors such as prostate cancer, lung cancer, non-small cell lung cancer (NSCLC), melanoma, lymphoma, breast cancer, head and neck cancer, renal cell carcinoma (RCC), ovarian cancer, kidney cancer, urinary bladder cancer, uterine cancer, cervical cancer, ovarian cancer, liver cancer, stomach cancer, colon cancer, rectal cancer, oral cavity cancer, pharynx cancer, pancreatic cancer, thyroid cancer, skin cancer, brain cancer are characterized to comprise HER2 expressing cells.

### BCMA related disorders

Within the meaning of the present invention, the subject matter described herein with an BCMA-binding antibody domain is suitable for therapeutic application in proliferative or malignant disorders associated with BCMA expression.

Hematological tumors such as Multiple Myeloma (MM), Waldenström's Macroglobulinemia, Chronic Lymphocytic Leukemia (CLL), Plasma Cell Leukemia, and Burkitt's Lymphoma are characterized by the presence of BCMA-expressing cells (B-cells).

Additionally, autoreactive B-cells in diseases such as Systemic Lupus Erythematosus (SLE) and Sjögren's Syndrome are also characterized by BCMA expression, presenting a potential therapeutic option for BCMA-directed CAR-T cell therapy.

### CD19 related disorders

Within the meaning of the present invention, the subject matter described herein with an CD19-binding antibody domain is suitable for therapeutic application in proliferative or malignant disorders associated with CD19 expression.

In particular, hematological tumors such as Acute Lymphoblastic Leukemia (ALL), Chronic Lymphocytic Leukemia (CLL), Diffuse Large B-Cell Lymphoma (DLBCL), Follicular Lymphoma (FL), Mantle Cell Lymphoma (MCL), Burkitt Lymphoma, Hairy Cell Leukemia (HCL), Marginal Zone Lymphoma (MZL), and Waldenström's Macroglobulinemia (WM) are characterized by the presence of CD19-expressing cells (B-cells).

The ubiquitous expression of CD19 on B-cells provides a strong rationale for developing targeted therapies in autoimmune diseases characterized by autoreactive B-cells. Thus CD19-targeted therapies hold significant promise for addressing autoimmune conditions such as Systemic Lupus Erythematosus (Guffroy et.al 2024), Rheumatoid Arthritis (RA), Myasthenia gravis (Haghikia et al 2023) Sjögren's Syndrome, and Multiple Sclerosis (Fischbach et al. 2024) offering a highly specific and effective treatment option.

The skilled person understands that the aforementioned HER2 (Erb2), BCMA and CD19 related disorders merely illustrate a preliminary list of options for respective targeting treatments and is certainly aware of further diseases that benefit from therapies specifically targeting HER2, BCMA, and CD19.

The following examples illustrate the invention with further details and serve to provide a better understanding of the invention but are not intended to limit the invention and its applicability in any way, especially not to the embodiments presented therein.

An aspect of the invention relates to a method of treating, preventing, or ameliorating a condition or disorder associated with the expression of an antigen, preferably a tumor antigen, in a subject, the method comprising administering to the subject an effective amount of the CAR as herein described, and/or the nucleic acid or recombinant vector as herein described, and/or the isolated mammalian cell or population thereof as herein described, and/or the pharmaceutical composition as herein described.

### Examples:

The examples described in further detail have no intention of limiting the scope of the invention and its applicability. They are intended to provide a better understanding of the invention and its broad applicability

### Example 1: Design and Preparation of DNA Constructs Demonstrating the Invention

To create DNA constructs (SEQ ID NOs: 11-16) encoding CARs with a human DR5 hinge and transmembrane domain (SEQ ID NOs: 5, 7, 9, 10) (**Figure 1A**) or the construct with only the DR5-based transmembrane domain (SEQ ID NO: 6 and 8) (**Figure 1B**), standard molecular biology methods were utilized. These included the chemical transformation of competent E. coli cells, plasmid DNA isolation, polymerase chain reaction (PCR), Gibson assembly, ligation, nucleic acid concentration measurement, agarose gel electrophoresis of DNA, extraction of DNA fragments from agarose gels, chemical DNA synthesis, digestion of DNA with restriction enzymes, digestion of plasmid vectors, ligation of DNA fragments, and large-scale plasmid DNA purification. These techniques are well-established and thoroughly documented in molecular biology manuals.

All plasmids, including complete and partial constructs, were introduced into E. coli NEB stable cells via chemical transformation. Plasmids intended for transfection into the Lenti-X 293T cell line were purified using the GeneJet Plasmid DNA Isolation Kit (Invitrogen).

### Example 2: Generation of CAR T Cells with a DR5 hinge and transmembrane domain

Viral Production: Lentiviral particles were generated by transiently transfecting plasmids into Lenti-X 293T cells. The cells were plated at a density of 5 × 10⁶ cells per plate. Twenty-four hours later, the cells were transfected with a mixture containing 15 µg of the pLVX-Puro vector (Addgene, 141395) encoding the target protein, 5µg of Additionally, pVSV-G (Addgene, 138479) and 12µg psPAX2 (Addgene, 12260) using polyethyleneimine MAX (PEI MAX). Forty-eight hours post-transfection, the supernatant containing the lentiviral particles was collected and filtered through a 0.45-µm filter. The filtered supernatant was then subjected to ultracentrifugation at 4°C for 2 hours at 100,000g using ultracentrifuge tubes. The resulting viral pellet was resuspended in phosphate-buffered saline (PBS) and stored at -80°C until required. CAR T Cell Construction: Primary human T cells were isolated from healthy donors following ethical and biosafety guidelines. Peripheral blood mononuclear cells (PBMCs) were separated from healthy donor buffy coats using Lymphoprep density gradient centrifugation. CD3+ T cells were purified through negative selection with the Pan T Cell Isolation Kit (Miltenyi Biotec) as per the manufacturer's instructions. Purified T cells were cultured in RPMI medium supplemented with 10% heat-inactivated fetal bovine serum (FBS), 25 µL/mL ImmunoCult Human CD3/CD28 T cell activator, and 50 U/mL human recombinant IL-2 for 48 hours before transduction.

T cells were transduced with lentiviral particles at a multiplicity of infection (MOI) of 15. After transduction, the T cells were cultured and expanded for at least 5-10 days in the presence of 50 U/mL human recombinant IL-2. The expression of the CAR construct was evaluated by flow cytometry, detecting Myc-tag expression on the cell surface. Primary T cells from multiple healthy donors were used in this procedure, with all samples handled in strict compliance with ethical and biosafety regulations.

### Example 3: In Vitro Cytotoxicity Assays

To evaluate the effectiveness of DR5-based CAR T cells with a modified hinge and transmembrane region (CAR of SEQ ID 5 and 9 of Figure 1A), their tumor-killing capacity was compared to second-generation CAR T cells (WT CAR of SEQ ID NO: 43 and 44) activity against CD19 and BCMA target antigens.

CAR T cells and unmodified T cells first undergo resting in a medium without human IL-2, followed by resuspension in the RPMI medium. Cancer cell lines expressing the target antigens were co-cultured with CAR T cells at specified effector-to-target (E: T) ratios 0,5:1. For CAR against CD19 Raji-Fluc cells have been used. For BCMA K562 Fluc cells had been used. The co-cultures were incubated under standard laboratory conditions for 24 hours. D-luciferin was added to the cultures to reach a final concentration of 150 µg/mL before measuring. Measurements were performed by bioluminescence imaging (BLI) on the IVIS Lumina Series III system. Processed and analyzed via Living Image 4.5.2 software. Specific target cell lysis percentages are calculated with this formula: % specific lysis = 100 × (spontaneous death ARV - test ARV)/(spontaneous death ARV - background ARV).

### Results:

The cytolytic efficiency of CAR T cells incorporating the DR5 domain was assessed in comparison to conventional CAR T cells featuring the CD8 transmembrane domain. When tested against CD19-positive RAJI cells, DR5 CAR T cells exhibited enhanced cytotoxicity, with the DR5 TMD contributing to increased killing potential. Similarly, when targeting BCMA, the DR5-based construct achieved higher lysis rates, underscoring the advantage of the DR5 adaptation. These results affirm that integrating the DR5 hinge and transmembrane domain strengthens the tumor-killing capacity of CAR T cells across multiple antigen targets.

### Example 4: Cytokine Secretion Analysis

To evaluate immune activation, cytokine secretion, IL-2, granzyme B, and IFN-γ were analyzed in CAR T cells incorporating the DR5 hinge and transmembrane domain. In order to compare DR5-based CAR (SEQ ID NO: 5) with a modified hinge and transmembrane region (Figure 1A) to wild-type CAR (WT CAR of SEQ ID NO: 43) constructs, supernatants from prior cytotoxicity assay co-cultures were collected by centrifuging at 1800 rpm for 5 minutes to separate cellular components. Human IL-2, IFN-γ, and Granzyme B levels in the supernatants were measured using ELISA kits from Thermo Fisher Scientific, following the manufacturer's protocol. Throughout the ELISA process, a HydroSpeed plate washer (Tecan) was utilized for washing steps, while absorbance was recorded using a SynergyMx microplate reader (BioTek), with cytokine concentrations determined from endpoint measurements.

### Results:

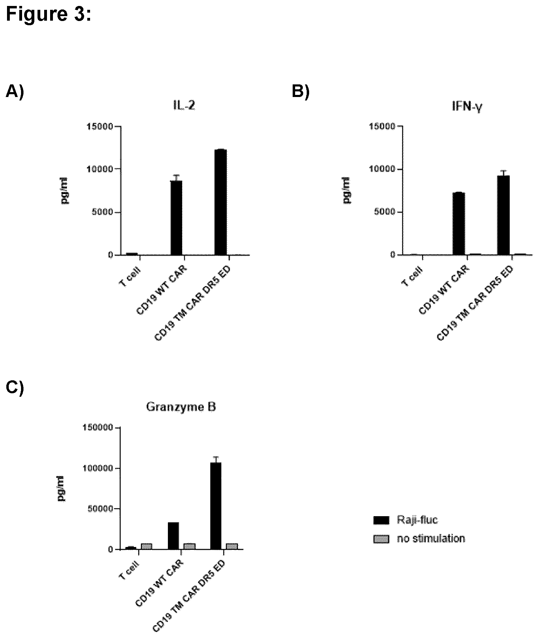

The cytokine secretion profiles for various target antigens are illustrated in Figure 3. When CD19-expressing RAJI cells were targeted, DR5-based CAR constructs exhibited greater IL-2, and Granzyme B IFN-γ production compared to WT CART cells. (Figure 3). This might explain the stronger tumor killing.

### Example 5: In Vitro Cytotoxicity Assays with different antigen densities

To evaluate the effectiveness of DR5-based CAR T cells (TM CAR DR5/ED of SEQ ID NO: 5 their tumor-killing capacity was compared to second-generation CAR T cells (WT CAR of SEQ ID NO: 43) activity against CD19 our construct. For the cocultures, we used a mixture of tumor cells exhibiting different antigen densities. The antigen densities were determined using flow cytometry and antibodies against CD19. MFI of CD19 was determined on Aurora cited. The higher the MFI the higher the Antigen density. We had three different tumor lines K562 cells with MFI of 1500 and 86000 and Raji-Fluc cells with an MFI of 300000. Cancer cell lines expressing the target antigens were co-cultured with CAR T cells at specified effector-to-target (E: T) ratios of 1:1 (Figure 4).

### Results:

In comparison to the WT CAR, the DR5-based CAR shows stronger killing in alloantigen densities, but most importantly the killing is much stronger, especially at the lower antigen densities (Figure 4).

### Example 6: In Vivo Tumor Elimination Using an Animal Model

The effectiveness of CAR T cells featuring a DR5 hinge and transmembrane domain was assessed in a live setting through a xenograft mouse model of human lymphoma. NGS mice (NOD scid gamma), raised in pathogen-free conditions, were housed under controlled environmental parameters, including a 12-hour light/dark cycle, relative humidity of 40-60%, and a temperature maintained between 20-24°C. The animals were kept in individually ventilated cages (IVC) and provided unrestricted access to standard laboratory chow and water. Each mouse arrived with a health certificate, and its microbiological status was verified following the FELASA-recommended Mouse Vivum panel (QM Diagnostics).

For this in vivo investigation, NGS mice aged 8 to 12 weeks were chosen. Tumor xenografts were induced intravenously administered 1 × 10⁶ RAJI-FLuc cells per mouse. Three days following tumor cell injection, each mouse received an intravenous dose of 5 × 10⁶ CAR T cells. The tumor burden was tracked weekly using bioluminescent imaging. Before imaging, the mice were injected intraperitoneally with D-luciferin (150 mg/kg; Sigma) and anesthetized with isoflurane inhalation. Imaging was performed 10 minutes after luciferin administration using the IVIS Lumina Series III system (PerkinElmer), with data acquisition and analysis conducted via Living Image 4.5.2 software (PerkinElmer), ensuring appropriate background subtraction.

After the experiment or upon reaching ethical endpoints, mice were humanely euthanized, and relevant data were collected.

### Results:

Mice treated with the WT CAR (SEQ ID NO: 43) and the DR5-based CAR (SEQ ID NO: 5) survived all in contrast with mice that received normal T cells (Figure 5A). In the not-treated group, 4 out of five mice had died by day 30. (Figure 5B) Tumor burden was quantified through total flux (photons/second), serving as a tumor progression marker. The DR5-based CAR T cells demonstrated superior tumor clearance over time, reflected by a decrease in bioluminescence compared to WT CAR. Among the tested constructs, DR5-based CAR exhibited the most effective tumor reduction (Figure 5B).

These results confirm that the introduction of the novel DR5 hinge and transmembrane domain CAR T cells enhances tumor suppression and improves survival outcomes in vivo.

### Example 7: Activation assay determination of CD69 expression

CAR T cells and unmodified T cells first undergo resting in a medium without human IL-2, followed by resuspension in the RPMI medium. Cancer cell lines expressing the target antigens were co-cultured with CAR T cells at specified effector-to-target (E: T) ratios 1:1. For CAR against CD19 Raji-Fluc cells have been used. The CD69 activation marker was measured after 1h, 2h, 4h and 30h. CAR-T cells without any stimulation were measured as well. Measuring was performed using antibodies against Myc-Tag (9B11) (found on our construct) Mouse monoclonal antibody (mAb) Alexa Fluor 488 Conjugate (Cell Signaling Technology). For CD69 detection Brilliant Violet 605^{™} antihuman CD69 Antibody (BioLegend) was used. To differentiate tumor cells CD19-APC human antibody was used (Miltenyi). Flow cytometry was performed on a Cytek Aurora Flow Cytometry System (Cytek Biosciences) and data was presented in Flowjo software.

### Results:

As expected the t-cells had the lowest CD69 expression. The WT CAR (SEQ ID NO: 43) had also shown lower expression to our DR5-based CAR (TM CAR DR5/ED of SEQ ID NO: 5), these results once again proved that the inclusion of our transmembrane and hinge domain significantly strengthens the activation leading to a faster and stronger tumor response (Figure 6).

### Example 8: Killing Assay: CAR constructs with DR5 transmembrane domain and DR5 hinge and transmembrane domain combinations

To evaluate the effectiveness of DR5-based CAR T cells (TM CAR DR5/ED and TM CAR DR5), their tumor-killing capacity was compared to second-generation CAR T cells (WT CAR) activity against CD19 target antigens.

CAR T cells and unmodified T cells first undergo resting in a medium without human IL-2, followed by resuspension in the RPMI medium. Cancer cell lines expressing the target antigens were co-cultured with CAR T cells at specified effector-to-target (E: T) ratios 0,5:1. For CAR against CD19 Raji-Fluc cells have been used. For BCMA K562 Fluc cells had been used. The co-cultures were incubated under standard laboratory conditions for 24 hours. D-luciferin was added to the cultures to reach a final concentration of 150 µg/mL before measuring. Measurements were performed by bioluminescence imaging (BLI) on the IVIS Lumina Series III system. Processed and analyzed via Living Image 4.5.2 software. Specific target cell lysis percentages are calculated with this formula: % specific lysis = 100 × (spontaneous death ARV - test ARV)/(spontaneous death ARV - background ARV).

### Results:

The cytolytic efficiency of CAR T cells incorporating the DR5-based transmembrane and hindege (black) (TM CAR DR5/ED), only the DR5 transmembrane domain (white) (TM CAR DR5) were assessed in comparison to conventional CAR T cells featuring the CD8 transmembrane domain. When tested against CD19-positive RAJI cells, DR5/ED CAR T cells incorporating both transmembrane and hinge domain exhibited enhanced cytotoxicity, whereas the TM CAR with only DR5 transmembrane domain did not outcompete the WT CAR. These results affirm that integrating the DR5 hinge and transmembrane domain strengthens the tumor-killing capacity of CAR T cells, and it also point to the fact that the transmembrane and hinge domain of DR5 do communicate and need to be used in tandem in order to function efficiently (Figure 7).

### Example 9 Sequences of the invention

| **SEQ ID NO:** | **DESCRIPTION** | **SEQUENCE** |
|---|---|---|
| 1 | DR5 hinge aminoacid sequence | CVHKESGTKHSGEVPAVEETVTSSPGTPASPCSL |
| 2 | DR5 hinge nucleic acid sequence | tgcgtgcacaaggagagcggcaccaagcacagcggcgaggtgcccgccgtggaggagaccgtgaccagcagccccggca cccccgccagcccctgcagcctg |
| 3 | DR5 transmembrane domin aminoacid sequence | SGIIIGVTVAAVVLIVAVFVCKSLL |
| 4 | DR5 transmembrane domain nucleic acid sequence | agcggcatcatcatcggcgtgaccgtggccgccgtggtgctgatcgtggccgtgttcgtgtgcaagagcctgctg |
| 5 | Exemplary anti-CD19 CAR amino acid sequence (CD8 leader_Myc-anti-CD19 scFv_DR5 ED hinge_DR5 TM_41BB_CD3ζ) | MALPVTALLLPLALLLHAARPEQKLISEEDLDIQMTQTTSSLSASLGDRVTISCRASQDISKYLNWYQ QKPDGTVKLLIYHTSRLHSGVPSRFSGSGSGTDYSLTISNLEQEDIATYFCQQGNTLPYTFGGGTKLEI TGGGGSGGGGSGGGGSEVKLQESGPGLVAPSQSLSVTCTVSGVSLPDYGVSWIRQPPRKGLEWLG VIWGSETTYYNSALKSRLTIIKDNSKSQVFLKMNSLQTDDTAIYYCAKHYYYGGSYAMDYWGQGTS VTVSSCVHKESGTKHSGEVPAVEETVTSSPGTPASPCSLSGIIIGVTVAAVVLIVAVFVCKSLLKRGRKK LLYIFKQPFMRPVQTTQEEDGCSCRFPEEEEGGCELRVKFSRSADAPAYQQGQNQLYNELNLGRRE EYDVLDKRRGRDPEMGGKPRRKNPQEGLYNELQKDKMAEAYSEIGMKGERRRGKGHDGLYQGLS TATKDTYDALHMQALPPR* |
| 6 | Exemplary anti-CD19 CAR amino acid sequence (CD8 leader_Myc-Tag_anti-CD19 scFv_CD8 hinge_DR5 TM_41BB_CD3ζ) | MALPVTALLLPLALLLHAARPEQKLISEEDLDIQMTQTTSSLSASLGDRVTISCRASQDISKYLNWYQ QKPDGTVKLLIYHTSRLHSGVPSRFSGSGSGTDYSLTISNLEQEDIATYFCQQGNTLPYTFGGGTKLEI TGGGGSGGGGSGGGGSEVKLQESGPGLVAPSQSLSVTCTVSGVSLPDYGVSWIRQPPRKGLEWLG VIWGSETTYYNSALKSRLTIIKDNSKSQVFLKMNSLQTDDTAIYYCAKHYYYGGSYAMDYWGQGTS VTVSSTTTPAPRPPTPAPTIASQPLSLRPEACRPAAGGAVHTRGLDFACDIYSGIIIGVTVAAVVLIVA VFVCKSLLKRGRKKLLYIFKQPFMRPVQTTQEEDGCSCRFPEEEEGGCELRVKFSRSADAPAYQQGQ NQLYNELNLGRREEYDVLDKRRGRDPEMGGKPRRKNPQEGLYNELQKDKMAEAYSEIGMKGERR RGKGHDGLYQGLSTATKDTYDALHMQALPPR* |
| 7 | Exemplary anti-CD19 CAR amino acid sequence (CD19 scFv_DR5 EDhinge_DR5 TM_41BB_CD3ζ) | MDIQMTQTTSSLSASLGDRVTISCRASQDISKYLNWYQQKPDGTVKLLIYHTSRLHSGVPSRFSGSG SGTDYSLTISNLEQEDIATYFCQQGNTLPYTFGGGTKLEITGGGGSGGGGSGGGGSEVKLQESGPGL VAPSQSLSVTCTVSGVSLPDYGVSWIRQPPRKGLEWLGVIWGSETTYYNSALKSRLTIIKDNSKSQVF LKMNSLQTDDTAIYYCAKHYYYGGSYAMDYWGQGTSVTVSSCVHKESGTKHSGEVPAVEETVTSS PGTPASPCSLSGIIIGVTVAAVVLIVAVFVCKSLLKRGRKKLLYIFKQPFMRPVQTTQEEDGCSCRFPEE EEGGCELRVKFSRSADAPAYQQGQNQLYNELNLGRREEYDVLDKRRGRDPEMGGKPRRKNPQEG LYNELQKDKMAEAYSEIGMKGERRRGKGHDGLYQGLSTATKDTYDALHMQALPPR* |
| 8 | Exemplary anti-CD19 CAR amino acid sequence (anti-CD19 scFv_CD8 hinge_DR5 TM_41BB_CD3ζ) | MDIQMTQTTSSLSASLGDRVTISCRASQDISKYLNWYQQKPDGTVKLLIYHTSRLHSGVPSRFSGSG SGTDYSLTISNLEQEDIATYFCQQGNTLPYTFGGGTKLEITGGGGSGGGGSGGGGSEVKLQESGPGL VAPSQSLSVTCTVSGVSLPDYGVSWIRQPPRKGLEWLGVIWGSETTYYNSALKSRLTIIKDNSKSQVF LKMNSLQTDDTAIYYCAKHYYYGGSYAMDYWGQGTSVTVSSTTTPAPRPPTPAPTIASQPLSLRPE ACRPAAGGAVHTRGLDFACDIYSGIIIGVTVAAVVLIVAVFVCKSLLKRGRKKLLYIFKQPFMRPVQTT QEEDGCSCRFPEEEEGGCELRVKFSRSADAPAYQQGQNQLYNELNLGRREEYDVLDKRRGRDPEM GGKPRRKNPQEGLYNELQKDKMAEAYSEIGMKGERRRGKGHDGLYQGLSTATKDTYDALHMQAL PPR* |
| 9 | Exemplary anti-BCMA CAR amino acid sequence (CD8 leader_Myc-Tag_anti-BCMA scFv_DR5 ED hinge_DR5 TM_41BB_CD3ζ) | MALPVTALLLPLALLLHAARPEQKLISEEDLQVQLVQSGAEVKKPGSSVKVSCKASGYTFTSYVMHW VRQAPGQGLEWMGYIIPYNDATKYNEKFKGRVTITADKSTSTAYMELSSLRSEDTAVYYCARYNYD GYFDVWGQGTLVTVSSGGGGSGGGGSGGGGSEIVLTQSPATLSLSPGERATLSCRASQSISDYLHW YQQKPGQAPRLLIYYASQSITGIPARFSGSGSGTDFTLTISSLEPEDFAVYYCQNGHSFPPTFGGGTKV EIKCVHKESGTKHSGEVPAVEETVTSSPGTPASPCSLSGIIIGVTVAAVVLIVAVFVCKSLLKRGRKKLL YIFKQPFMRPVQTTQEEDGCSCRFPEEEEGGCELRVKFSRSADAPAYQQGQNQLYNELNLGRREEY DVLDKRRGRDPEMGGKPRRKNPQEGLYNELQKDKMAEAYSEIGMKGERRRGKGHDGLYQGLST ATKDTYDALHMQALPPR* |
| 10 | Exemplary anti-BCMA CAR amino acid sequence (anti-BCMA scFv_DR5 ED hinge_DR5 TM_41BB_CD3ζ) | MQVQLVQSGAEVKKPGSSVKVSCKASGYTFTSYVMHWVRQAPGQGLEWMGYIIPYNDATKYNE KFKGRVTITADKSTSTAYMELSSLRSEDTAVYYCARYNYDGYFDVWGQGTLVTVSSGGGGSGGGG SGGGGSEIVLTQSPATLSLSPGERATLSCRASQSISDYLHWYQQKPGQAPRLLIYYASQSITGIPARFS GSGSGTDFTLTISSLEPEDFAVYYCQNGHSFPPTFGGGTKVEIKCVHKESGTKHSGEVPAVEETVTSS PGTPASPCSLSGIIIGVTVAAVVLIVAVFVCKSLLKRGRKKLLYIFKQPFMRPVQTTQEEDGCSCRFPEE EEGGCELRVKFSRSADAPAYQQGQNQLYNELNLGRREEYDVLDKRRGRDPEMGGKPRRKNPQEG LYNELQKDKMAEAYSEIGMKGERRRGKGHDGLYQGLSTATKDTYDALHMQALPPR* |
| 11 | Exemplary anti-CD19 CAR nucleic acid sequence (CD8 leader_Myc-anti-CD19 scFv_DR5 ED hinge_DR5 TM_41BB_CD3ζ) | atggccctgcctgtgacagctctgctgctgcctctggccctgctgctgcatgccgccagacccgagcagaagctgatcagcga ggaggacctggacattcaaatgactcaaacaacttcatctttgtctgcaagcctgggagaccgcgtgacaataagctgccga gccagtcaagatatcagcaaatatctcaattggtatcagcaaaagcctgatgggactgttaaattgttgatataccatacgag tcgcctccacagcggagttccttctaggttttctggatcaggctccgggaccgactactccctcacgattagcaatcttgagca ggaggatatagccacatatttttgtcaacaaggaaacacacttccctacaccttcggtgggggaacgaaattggaaattactg gtggcggcgggtctggaggtggcggtagtggaggcgggggctctgaagtcaagcttcaagaatcaggtccgggacttgtag cgccaagtcagtctctttccgtaacttgtaccgttagtggggtttccttgccagattacggagtaagttggattcgccaaccgcc cagaaagggcttggaatggttgggtgttatctggggaagcgaaacaacatactataacagcgcactcaagtctcgcctcacg attattaaagataactctaaatctcaggtgttcctcaaaatgaactcattgcaaacggacgacactgccatctattactgtgca aaacactattactacggcggctcttacgctatggattactggggccagggaacttcagtgacagtttctagttgcgtgcacaa ggagagcggcaccaagcacagcggcgaggtgcccgccgtggaggagaccgtgaccagcagccccggcacccccgccagc ccctgcagcctgagcggcatcatcatcggcgtgaccgtggccgccgtggtgctgatcgtggccgtgttcgtgtgcaagagcct gctgaagcggggcagaaagaaactgctgtacatctttaagcagcccttcatgcggcccgtgcagaccacccaggaagagga cggctgctcctgcagattccccgaggaagaagaaggcggctgcgagctgagagtgaagttcagcagatccgccgacgcccc tgcctaccagcagggacagaaccagctgtacaacgagctgaacctgggcagacgggaagagtacgacgtgctggacaagc ggagaggccgggatcctgaaatgggcggcaagcccagacggaagaacccccaggaaggcctgtataacgaactgcagaa agacaagatggccgaggcctacagcgagatcggaatgaagggcgagcggagaagaggcaagggccacgatggcctgtac cagggcctgagcaccgccaccaaggacacctatgacgccctgcacatgcaggctttgccgcctagataa |
| 12 | Exemplary anti-CD19 CAR nucleic acid sequence (CD8 leader_Myc-Tag_anti-CD19 scFv_CD8 hinge_DR5 TM_41BB_CD3ζ) | atggccctgcctgtgacagctctgctgctgcctctggccctgctgctgcatgccgccagacccgagcagaagctgatcagcga ggaggacctggacattcaaatgactcaaacaacttcatctttgtctgcaagcctgggagaccgcgtgacaataagctgccga gccagtcaagatatcagcaaatatctcaattggtatcagcaaaagcctgatggactgttaaattgttgatataccatacgag tcgcctccacagcggagttccttctaggttttctggatcaggctccgggaccgactactccctcacgattagcaatcttgagca ggaggatatagccacatatttttgtcaacaaggaaacacacttccctacaccttcggtggggggaacgaaattggaaattactg gtggcggcgggtctggaggtggcggtagtggaggcgggggctctgaagtcaagcttcaagaatcaggtccgggacttgtag cgccaagtcagtctctttccgtaacttgtaccgttagtggggtttccttgccagattacggagtaagttggattcgccaaccgcc cagaaagggcttggaatggttgggtgttatctggggaagcgaaacaacatactataacagcgcactcaagtctcgcctcacg attattaaagataactctaaatctcaggtgttcctcaaaatgaactcattgcaaacggacgacactgccatctattactgtgca aaacactattactacggcggctcttacgctatggattactggggccagggaacttcagtgacagtttctagtacaaccacccct gcccctagacctccaaccccagcccctacaatcgccagccagcctctgtctctgaggcccgaggcttgtagaccagctgctgg cggagccgtgcacaccagaggactggatttcgcctgcgacatctacagcggcatcatcatcggcgtgaccgtggccgccgtg gtgctgatcgtggccgtgttcgtgtgcaagagcctgctgaagcggggcagaaagaaactgctgtacatctttaagcagccctt catgcggcccgtgcagaccacccaggaagaggacggctgctcctgcagattccccgaggaagaagaaggcggctgcgagc tgagagtgaagttcagcagatccgccgacgcccctgcctaccagcagggacagaaccagctgtacaacgagctgaacctgg gcagacgggaagagtacgacgtgctggacaagcggagaggccgggatcctgaaatgggcggcaagcccagacggaagaa cccccaggaaggcctgtataacgaactgcagaaagacaagatggccgaggcctacagcgagatcggaatgaagggcgag cggagaagaggcaagggccacgatggcctgtaccagggcctgagcaccgccaccaaggacacctatgacgccctgcacat gcaggctttgccgcctagataa |
| 13 | Exemplary anti-CD19 CAR nucleic acid sequence (anti-CD19 scFv_DR5 ED hinge_DR5 TM_41BB_CD3ζ) | atggacattcaaatgactcaaacaacttcatctttgtctgcaagcctgggagaccgcgtgacaataagctgccgagccagtca agatatcagcaaatatctcaattggtatcagcaaaagcctgatgggactgttaaattgttgatataccatacgagtcgcctcc acagcggagttccttctaggttttctggatcaggctccgggaccgactactccctcacgattagcaatcttgagcaggaggata tagccacatatttttgtcaacaaggaaacacacttccctacaccttcggtgggggaacgaaattggaaattactggtggcggc gggtctggaggtggcggtagtggaggcgggggctctgaagtcaagcttcaagaatcaggtccgggacttgtagcgccaagtc agtctctttccgtaacttgtaccgttagtggggtttccttgccagattacggagtaagttggattcgccaaccgcccagaaagg gcttggaatggttgggtgttatctggggaagcgaaacaacatactataacagcgcactcaagtctcgcctcacgattattaaa gataactctaaatctcaggtgttcctcaaaatgaactcattgcaaacggacgacactgccatctattactgtgcaaaacactat tactacggcggctcttacgctatggattactggggccagggaacttcagtgacagtttctagttgcgtgcacaaggagagcgg caccaagcacagcggcgaggtgcccgccgtggaggagaccgtgaccagcagccccggcacccccgccagcccctgcagcc tgagcggcatcatcatcggcgtgaccgtggccgccgtggtgctgatcgtggccgtgttcgtgtgcaagagcctgctgaagcgg ggcagaaagaaactgctgtacatctttaagcagcccttcatgcggcccgtgcagaccacccaggaagaggacggctgctcct gcagattccccgaggaagaagaaggcggctgcgagctgagagtgaagttcagcagatccgccgacgcccctgcctaccagc agggacagaaccagctgtacaacgagctgaacctgggcagacgggaagagtacgacgtgctggacaagcggagaggccg ggatcctgaaatgggcggcaagcccagacggaagaacccccaggaaggcctgtataacgaactgcagaaagacaagatg gccgaggcctacagcgagatcggaatgaagggcgagcggagaagaggcaagggccacgatggcctgtaccagggcctga gcaccgccaccaaggacacctatgacgccctgcacatgcaggctttgccgcctagataa |
| 14 | Exemplary anti-CD19 CAR nucleic acid sequence (anti-CD19 scFv_CD8 hinge_DR5 TM_41BB_CD3ζ) | atggacattcaaatgactcaaacaacttcatctttgtctgcaagcctgggagaccgcgtgacaataagctgccgagccagtca agatatcagcaaatatctcaattggtatcagcaaaagcctgatgggactgttaaattgttgatataccatacgagtcgcctcc acagcggagttccttctaggttttctggatcaggctccgggaccgactactccctcacgattagcaatcttgagcaggaggata tagccacatatttttgtcaacaaggaaacacacttccctacaccttcggtggggggaacgaaattggaaattactggtggcggc gggtctggaggtggcggtagtggaggcgggggctctgaagtcaagcttcaagaatcaggtccgggacttgtagcgccaagtc agtctctttccgtaacttgtaccgttagtggggtttccttgccagattacggagtaagttggattcgccaaccgcccagaaagg gcttggaatggttgggtgttatctggggaagcgaaacaacatactataacagcgcactcaagtctcgcctcacgattattaaa gataactctaaatctcaggtgttcctcaaaatgaactcattgcaaacggacgacactgccatctattactgtgcaaaacactat tactacggcggctcttacgctatggattactggggccagggaacttcagtgacagtttctagtacaaccacccctgcccctaga cctccaaccccagcccctacaatcgccagccagcctctgtctctgaggcccgaggcttgtagaccagctgctggcggagccgt gcacaccagaggactggatttcgcctgcgacatctacagcggcatcatcatcggcgtgaccgtggccgccgtggtgctgatcg tggccgtgttcgtgtgcaagagcctgctgaagcggggcagaaagaaactgctgtacatctttaagcagcccttcatgcggccc gtgcagaccacccaggaagaggacggctgctcctgcagattccccgaggaagaagaaggcggctgcgagctgagagtgaa gttcagcagatccgccgacgcccctgcctaccagcagggacagaaccagctgtacaacgagctgaacctgggcagacggga agagtacgacgtgctggacaagcggagaggccgggatcctgaaatgggcggcaagcccagacggaagaacccccaggaa ggcctgtataacgaactgcagaaagacaagatggccgaggcctacagcgagatcggaatgaagggcgagcggagaagag gcaagggccacgatggcctgtaccagggcctgagcaccgccaccaaggacacctatgacgccctgcacatgcaggctttgc cgcctagataa |
| 15 | Exemplary anti-BCMA nucleic acid sequence (CD8 leader_Myc-Tag_anti-BCMA scFv_DR5 ED hinge_DR5 TM_41BB_CD3ζ) | atggccctgcctgtgacagctctgctgctgcctctggccctgctgctgcatgccgccagacccgagcagaagctgatcagcga ggaggacctgcaggtgcagctggtgcagagcggcgcggaagtgaaaaaaccgggcagcagcgtgaaagtgagctgcaaa gcgagcggctatacctttaccagctatgtgatgcattgggtgcgccaggcgccgggccagggcctggaatggatgggctatat tattccgtataacgatgcgaccaaatataacgaaaaatttaaaggccgcgtgaccattaccgcggataaaagcaccagcac cgcgtatatggaactgagcagcctgcgcagcgaagataccgcggtgtattattgcgcgcgctataactatgatggctattttg atgtgtggggccagggcaccctggtgaccgtgagcagcggtggcggcgggtctggaggtggcggtagtggaggcgggggct ctgaaattgtgctgacccagagcccggcgaccctgagcctgagcccgggcgaacgcgcgaccctgagctgccgcgcgagcc agagcattagcgattatctgcattggtatcagcagaaaccgggccaggcgccgcgcctgctgatttattatgcgagccagagc attaccggcattccggcgcgctttagcggcagcggcagcggcaccgattttaccctgaccattagcagcctggaaccggaag attttgcggtgtattattgccagaacggccatagctttccgccgacctttggcggcggcaccaaagtggaaattaaatgcgtgc acaaggagagcggcaccaagcacagcggcgaggtgcccgccgtggaggagaccgtgaccagcagccccggcacccccgc cagcccctgcagcctgagcggcatcatcatcggcgtgaccgtggccgccgtggtgctgatcgtggccgtgttcgtgtgcaaga gcctgctgaagcggggcagaaagaaactgctgtacatctttaagcagcccttcatgcggcccgtgcagaccacccaggaag aggacggctgctcctgcagattccccgaggaagaagaaggcggctgcgagctgagagtgaagttcagcagatccgccgacg cccctgcctaccagcagggacagaaccagctgtacaacgagctgaacctgggcagacgggaagagtacgacgtgctggac aagcggagaggccgggatcctgaaatgggcggcaagcccagacggaagaacccccaggaaggcctgtataacgaactgc agaaagacaagatggccgaggcctacagcgagatcggaatgaagggcgagcggagaagaggcaagggccacgatggcc tgtaccagggcctgagcaccgccaccaaggacacctatgacgccctgcacatgcaggctttgccgcctagataa |
| 16 | Exemplary anti-BCMA nucleic acid sequence (anti-BCMA scFv_DR5 ED hinge_DR5 TM_41BB_CD3ζ) | atgcaggtgcagctggtgcagagcggcgcggaagtgaaaaaaccgggcagcagcgtgaaagtgagctgcaaagcgagcg gctatacctttaccagctatgtgatgcattgggtgcgccaggcgccgggccagggcctggaatggatgggctatattattccgt ataacgatgcgaccaaatataacgaaaaatttaaaggccgcgtgaccattaccgcggataaaagcaccagcaccgcgtata tggaactgagcagcctgcgcagcgaagataccgcggtgtattattgcgcgcgctataactatgatggctattttgatgtgtggg gccagggcaccctggtgaccgtgagcagcggtggcggcgggtctggaggtggcggtagtggaggcgggggctctgaaattg tgctgacccagagcccggcgaccctgagcctgagcccgggcgaacgcgcgaccctgagctgccgcgcgagccagagcatta gcgattatctgcattggtatcagcagaaaccgggccaggcgccgcgcctgctgatttattatgcgagccagagcattaccggc attccggcgcgctttagcggcagcggcagcggcaccgattttaccctgaccattagcagcctggaaccggaagattttgcggt gtattattgccagaacggccatagctttccgccgacctttggcggcggcaccaaagtggaaattaaatgcgtgcacaaggag agcggcaccaagcacagcggcgaggtgcccgccgtggaggagaccgtgaccagcagccccggcacccccgccagcccctg cagcctgagcggcatcatcatcggcgtgaccgtggccgccgtggtgctgatcgtggccgtgttcgtgtgcaagagcctgctga agcggggcagaaagaaactgctgtacatctttaagcagcccttcatgcggcccgtgcagaccacccaggaagaggacggct gctcctgcagattccccgaggaagaagaaggcggctgcgagctgagagtgaagttcagcagatccgccgacgcccctgcct accagcagggacagaaccagctgtacaacgagctgaacctgggcagacgggaagagtacgacgtgctggacaagcggag aggccgggatcctgaaatgggcggcaagcccagacggaagaacccccaggaaggcctgtataacgaactgcagaaagac aagatggccgaggcctacagcgagatcggaatgaagggcgagcggagaagaggcaagggccacgatggcctgtaccagg gcctgagcaccgccaccaaggacacctatgacgccctgcacatgcaggctttgccgcctagataa |
| 17 | Human CD19 (UniProt: P15391-1) | |
| 18 | Human CD19 (UniProt: P15391-2) | MPPPRLLFFLLFLTPMEVRPEEPLVVKVEEGDNAVLQCLKGTSDGPTQQLTWSRESPLKPFLKLSLGL PGLGIHMRPLAIWLFIFNVSQQMGGFYLCQPGPPSEKAWQPGWTVNVEGSGELFRWNVSDLGG LGCGLKNRSSEGPSSPSGKLMSPKLYVWAKDRPEIWEGEPPCLPPRDSLNQSLSQDLTMAPGSTL WLSCGVPPDSVSRGPLSWTHVHPKGPKSLLSLELKDDRPARDMWVMETGLLLPRATAQDAGKYY CHRGNLTMSFHLEITARPVLWHWLLRTGGWKVSAVTLAYLIFCLCSLVGILHLQRALVLRRKRKRM TDPTRRFFKVTPPPGSGPQNQYGNVLSLPTPTSGLGRAQRWAAGLGGTAPSYGNPSSDVQADGA LGSRSPPGVGPEEEEGEGYEEPDSEEDSEFYENDSNLGQDQLSQDGSGYENPEDEPLGPEDEDSFS NAESYENEDEELTQPVARTMDFLSPHGSAWDPSREATSLAGSQSYEDMRGILYAAPQLRSIRGQP GPNHEEDADSYENMDNPDGPDPAWGGGGRMGTWSTR |
| 19 | Human CD19 (UniProt: P15391-1) signal domain | MPPPRLLFFLLFLTPMEVR |
| 20 | Human CD19 (UniProt: P15391-1) extracellular domain | PEEPLVVKVEEGDNAVLQCLKGTSDGPTQQLTWSRESPLKPFLKLSLGLPGLGIHMRPLAIWLFIFNV SQQMGGFYLCQPGPPSEKAWQPGWTVNVEGSGELFRWNVSDLGGLGCGLKNRSSEGPSSPSGK LMSPKLYVWAKDRPEIWEGEPPCLPPRDSLNQSLSQDLTMAPGSTLWLSCGVPPDSVSRGPLSWT HVHPKGPKSLLSLELKDDRPARDMWVMETGLLLPRATAQDAGKYYCHRGNLTMSFHLEITARPVL WHWLLRTGGWK |
| 21 | Human CD19 (UniProt: P15391-1) transmembrane domain | VSAVTLAYLI FCLCSLVGI LH L |
| 22 | Human CD19 (UniProt: P15391-1) cytoplasmic domain | QRALVLRRKRKRMTDPTRRFFKVTPPPGSGPQNQYGNVLSLPTPTSGLGRAQRWAAGLGGTAPSY GNPSSDVQADGALGSRSPPGVGPEEEEGEGYEEPDSEEDSEFYENDSNLGQDQLSQDGSGYENPE DEPLGPEDEDSFSNAESYENEDEELTQPVARTMDFLSPHGSAWDPSREATSLGSQSYEDMRGILYA APQLRSIRGQPGPNHEEDADSYENMDNPDGPDPAWGGGGRMGTWSTR |
| 23 | Human BCMA (UniProt: Q02223-1) | MLQMAGQCSQNEYFDSLLHACIPCQLRCSSNTPPLTCQRYCNASVTNSVKGTNAILWTCLGLSLIIS LAVFVLMFLLRKINSEPLKDEFKNTGSGLLGMANIDLEKSRTGDEIILPRGLEYTVEECTCEDCIKSKPK VDSDHCFPLPAMEEGATILVTTKTNDYCKSLPAALSATEIEKSISAR |
| 24 | Human BCMA (UniProt: Q02223-2) | MLQMAGQCSQNEYFDSLLHACIPCQLRCSSNTPPLTCQRYCNARSGLLGMANIDLEKSRTGDEIILP RGLEYTVEECTCEDCIKSKPKVDSDHCFPLPAMEEGATILVTTKTNDYCKSLPAALSATEIEKSISAR |
| 25 | Human BCMA (UniProt: Q02223-1) extracellular domain | MLQMAGQCSQNEYFDSLLHACIPCQLRCSSNTPPLTCQRYCNASVTNSVKGTNA |
| 26 | Human BCMA (UniProt: Q02223-1) transmembrane domain | ILWTCLGLSLIISLAVFVLMFLL |
| 27 | Human BCMA (UniProt: Q02223-1) cytoplasmic domain | RKINSEPLKDEFKNTGSGLLGMANIDLEKSRTGDEIILPRGLEYTVEECTCEDCIKSKPKVDSDHCFPLP AMEEGATILVTTKTNDYCKSLPAALSATEIEKSISAR |
| 28 | Linker sequence 1 | GGGS |
| 29 | Linker sequence 2 | GGGGS |
| 30 | Linker sequence 3 | GGGSG |
| 31 | Linker sequence 4 | GGGGSG |
| 32 | Linker sequence 5 | GGGGSGGGGSGGGGS |
| 33 | Linker sequence 6 | GGGGSGGGGSGGGGSGGGGS |
| 34 | Linker sequence 7 | GGGGSGGGGSGGGGSGGGGSGGGGSGGGGS |
| 35 | CD8α hinge | TTTPAPRPPTPAPTIASQPLSLRPEACRPAAGGAVHTRGLDFACDIY |
| 36 | CD8α transmembrane domain | IWAPLAGTCGVLLLSLVITLYC |
| 37 | ITAM consensus | YXXL/I |
| | | wherein X = any amino acid |
| 38 | Larger ITAM consensus | YXXL/I(X)₆₋₈YXXL/I |
| | | wherein X = any amino acid |
| 39 | Human 4-1BB intracellular domain | KRGRKKLLYIFKQPFMRPVQTTQEEDGCSCRFPEEEEGGCEL |
| 40 | Human CD3ζ intracellular domain | RVKFSRSADAPAYQQGQNQLYNELNLGRREEYDVLDKRRGRDPEMGGKPRRKNPQEGLYNELQK DKMAEAYSEIGMKGERRRGKGHDGLYQGLSTATKDTYDALHMQALPPR |
| 41 | Myc tag | EQKLISEEDL |
| 42 | CD8 leader | ALPVTALLLPLALLLHAARP |
| 43 | Control anti-CD19 CAR amino acid sequence (CD8 leader_Myc-Tag_anti-CD19 scFv_CD8 hinge_CD8 TM_41BB_CD3ζ) | ALPVTALLLPLALLLHAARPEQKLISEEDLDIQMTQTTSSLSASLGDRVTISCRASQDISKYLNWYQQK PDGTVKLLIYHTSRLHSGVPSRFSGSGSGTDYSLTISNLEQEDIATYFCQQGNTLPYTFGGGTKLEITG GGGSGGGGSGGGGSEVKLQESGPGLVAPSQSLSVTCTVSGVSLPDYGVSWIRQPPRKGLEWLGVI WGSETTYYNSALKSRLTIIKDNSKSQVFLKMNSLQTDDTAIYYCAKHYYYGGSYAMDYWGQGTSVT VSSTTTPAPRPPTPAPTIASQPLSLRPEACRPAAGGAVHTRGLDFACDIYIWAPLAGTCGVLLLSLVIT LYCKRGRKKLLYIFKQPFMRPVQTTQEEDGCSCRFPEEEEGGCELRVKFSRSADAPAYQQGQNQLY NELNLGRREEYDVLDKRRGRDPEMGGKPRRKNPQEGLYNELQKDKMAEAYSEIGMKGERRRGKG HDGLYQGLSTATKDTYDALHMQALPPR |
| 44 | Control anti-BCMA CAR amino acid sequence (CD8 leader_Myc-Tag_anti-BCMA scFv_CD8 hinge_CD8 TM_41BB_CD3ζ) | ALPVTALLLPLALLLHAARPEQKLISEEDLQVQLVQSGAEVKKPGSSVKVSCKASGYTFTSYVMHWV RQAPGQGLEWMGYIIPYNDATKYNEKFKGRVTITADKSTSTAYMELSSLRSEDTAVYYCARYNYDG YFDVWGQGTLVTVSSGGGGSGGGGSGGGGSEIVLTQSPATLSLSPGERATLSCRASQSISDYLHWY QQKPGQAPRLLIYYASQSITGIPARFSGSGSGTDFTLTISSLEPEDFAVYYCQNGHSFPPTFGGGTKVE IKTTTPAPRPPTPAPTIASQPLSLRPEACRPAAGGAVHTRGLDFACDIYIWAPLAGTCGVLLLSLVITLY CKRGRKKLLYIFKQPFMRPVQTTQEEDGCSCRFPEEEEGGCELRVKFSRSADAPAYQQGQNQLYNE LNLGRREEYDVLDKRRGRDPEMGGKPRRKNPQEGLYNELQKDKMAEAYSEIGMKGERRRGKGHD GLYQGLSTATKDTYDALHMQALPPR |
| 45 | Anti-CD19 VH | EVKLQESGPGLVAPSQSLSVTCTVSGVSLPDYGVSWIRQPPRKGLEWLGVIWGSETTYYNSALKSRL TIIKDNSKSQVFLKMNSLQTDDTAIYYCAKHYYYGGSYAMDYWGQGTSVTVSS |
| 46 | Anti-CD19 HC-CDR1 | GVSLPDYG |
| 47 | Anti-CD19 HC-CDR2 | IWGSETT |
| 48 | Anti-CD19 HC-CDR3 | AKHYYYGGSYAMDY |
| 49 | Anti-CD19 VL | DIQMTQTTSSLSASLGDRVTISCRASQDISKYLNWYQQKPDGTVKLLIYHTSRLHSGVPSRFSGSGSG TDYSLTISNLEQEDIATYFCQQGNTLPYTFGGGTKLEIT |
| 50 | Anti-CD19 LC-CDR1 | QDISKY |
| 51 | Anti-CD19 LC-CDR2 | HTS |
| 52 | Anti-CD19 LC-CDR3 | QQGNTLPYT |
| 53 | Anti-CD19 scFv | DIQMTQTTSSLSASLGDRVTISCRASQDISKYLNWYQQKPDGTVKLLIYHTSRLHSGVPSRFSGSGSG TDYSLTISNLEQEDIATYFCQQGNTLPYTFGGGTKLEITGGGGSGGGGSGGGGSEVKLQESGPGLVA PSQSLSVTCTVSGVSLPDYGVSWIRQPPRKGLEWLGVIWGSETTYYNSALKSRLTIIKDNSKSQVFLK MNSLQTDDTAIYYCAKHYYYGGSYAMDYWGQGTSVTVSS |
| 54 | Anti-BCMA VH | QVQLVQSGAEVKKPGSSVKVSCKASGYTFTSYVMHWVRQAPGQGLEWMGYIIPYNDATKYNEKF KGRVTITADKSTSTAYMELSSLRSEDTAVYYCARYNYDGYFDVWGQGTLVTVSS |
| 55 | Anti-BCMA HC-CDR1 | GYTFTSYV |
| 56 | Anti-BCMA HC-CDR2 | IIPYNDAT |
| 57 | Anti-BCMA HC-CDR3 | ARYNYDGYFDV |
| 58 | Anti-BCMA VL | EIVLTQSPATLSLSPGERATLSCRASQSISDYLHWYQQKPGQAPRLLIYYASQSITGIPARFSGSGSGT DFTLTISSLEPEDFAVYYCQNGHSFPPTFGGGTKVEIKGSG |
| 59 | Anti-BCMA LC-CDR1 | QSISDY |
| 60 | Anti-BCMA LC-CDR2 | YAS |
| 61 | Anti-BCMA LC-CDR3 | QNGHSFPPT |
| 62 | Anti-BCMA scFv | QVQLVQSGAEVKKPGSSVKVSCKASGYTFTSYVMHWVRQAPGQGLEWMGYIIPYNDATKYNEKF KGRVTITADKSTSTAYMELSSLRSEDTAVYYCARYNYDGYFDVWGQGTLVTVSSGGGGSGGGGSG GGGSEIVLTQSPATLSLSPGERATLSCRASQSISDYLHWYQQKPGQAPRLLIYYASQSITGIPARFSGS GSGTDFTLTISSLEPEDFAVYYCQNGHSFPPTFGGGTKVEIKGSG |
| 63 | sp\|O14763\|TR10B_H UMAN Tumor necrosis factor receptor superfamily member 10B OS=Homo sapiens OX=9606 GN=TNFRSF10B PE=1 SV=2 | MEQRGQNAPAASGARKRHGPGPREARGARPGPRVPKTLVLVVAAVLLLVSAESALITQQDLAPQQ RAAPQQKRSSPSEGLCPPGHHISEDGRDCISCKYGQDYSTHWNDLLFCLRCTRCDSGEVELSPCTTT RNTVCQCEEGTFREEDSPEMCRKCRTGCPRGMVKVGDCTPWSDIECVHKESGTKHSGEVPAVEET VTSSPGTPASPCSLSGIIIGVTVAAVVLIVAVFVCKSLLWKKVLPYLKGICSGGGGDPERVDRSSQRPG AEDNVLNEIVSILQPTQVPEQEMEVQEPAEPTGVNMLSPGESEHLLEPAEAERSQRRRLLVPANEG DPTETLRQCFDDFADLVPFDSWEPLMRKLGLMDNEIKVAKAEAAGHRDTLYTMLIKWVNKTGRD ASVHTLLDALETLGERLAKQKIEDHLLSSGKFMYLEGNADSAMS |
| 64 | Human CD28 intracellular domain | RSKRSRLLHSDYMNMTPRRPGPTRKHYQPYAPPRDFAAYRS |
| 65 | Human CD28 intracellular domain | cggagcaagagatccagactgctgcactccgactacatgaacatgacccccagacggccaggccccacccggaaacactat cagccttacgcccctcccagagacttcgccgcctaccggtcc |
| 66 | Anti-ErbB2 scFv | DIQMTQSPSSLSASVGDRVTITCRASQDVNTAVAWYQQKPGKAPKLLIYSASFLYSGVPSRFSGSRS GTDFTLTISSLQPEDFATYYCQQHYTTPPTFGQGTKVEIKRTGSTSGSGKPGSGEGSEVQLVESGGGL VQPGGSLRLSCAASGFNIKDTYIHWVRQAPGKGLEWVARIYPTNGYTRYADSVKGRFTISADTSKN TAYLQMNSLRAEDTAVYYCSRWGGDGFYAMDVWGQGTLVTVSS |
| 67 | Anti-ErbB2 scFv | gacatccagatgacccagagtcccagcagtctgagcgctagtgtaggcgaccgcgttaccatcacatgcagagcatctcaag atgtgaatacagcagttgcgtggtatcaacagaaacctggtaaagcccctaaactgctcatctatagtgcgtcctttctgtata gtggcgtcccctcacgcttcagcggctcacgctctgggacagactttaccttgaccatctcttcccttcagcctgaggactttgc cacctattattgtcagcaacattacaccacaccaccaacattcgggcaggggacgaaagtagagataaaaaggacgggctc tacaagtgggtctggaaaaccgggctcaggggaaggatctgaggtgcaactcgttgaaagtggcggcggattggtccagcc gggggggatccttgcgccttagctgtgctgcatcaggatttaatatcaaggacacttacatccactgggtaaggcaagcccctg gaaaggggcttgagtgggtagcccggatctaccctacaaacggttatacccgctacgcggacagcgtgaaaggccgcttcac aatttcagccgatacttctaaaaacactgcgtacctccaaatgaactcactgcgggcggaagatactgcagtttattactgta gcagatggggtggggatgggttttatgccatggatgtatgggggcagggaacacttgtaaccgtttctagc |

### References

Awasthi, Rakesh, Harald J. Maier, Jie Zhang, and Stephen Lim. 2023. "Kymriah® (Tisagenlecleucel) - An Overview of the Clinical Development Journey of the First Approved CAR-T Therapy." Human Vaccines & Immunotherapeutics 19 (1): 2210046. https://doi.org/10.1080/21645515.2023.2210046.
Cibrián, Danay, and Francisco Sánchez-Madrid. 2017. "CD69: From Activation Marker to Metabolic Gatekeeper." European Journal of Immunology 47 (6): 946-53. https://doi.org/10.1002/eji.201646837.
Elazar, Assaf, Nicholas J Chandler, Ashleigh S Davey, Jonathan Y Weinstein, Julie V Nguyen, Raphael Trenker, Ryan S Cross, et al. 2022. "De Novo-Designed Transmembrane Domains Tune Engineered Receptor Functions." eLife 11 (May):e75660. https://doi.org/10.7554/eLife.75660.
Jayaraman, Jayapriya, Michael P. Mellody, Andrew J. Hou, Ruchi P. Desai, Audrey W. Fung, An Huynh Thuy Pham, Yvonne Y. Chen, and Weian Zhao. 2020. "CAR-T Design: Elements and Their Synergistic Function." EBioMedicine 58 (August): 102931. https://doi.org/10.1016/j.ebiom.2020.102931.
June, Carl H., and Michel Sadelain. 2018. "Chimeric Antigen Receptor Therapy." New England Journal of Medicine 379 (1): 64-73. https:/Idoi.org/10.1056/NEJMra1706169.
Li, Nan, Alex Quan, Dan Li, Jiajia Pan, Hua Ren, Gerard Hoeltzel, Natalia De Val, et al. 2023. "The IgG4 Hinge with CD28 Transmembrane Domain Improves VHH-Based CAR T Cells Targeting a Membrane-Distal Epitope of GPC1 in Pancreatic Cancer." Nature Communications 14 (1): 1986. https://doi.org/10.1038/s41467-023-37616-4.
Mueller, Benjamin K., Sabareesh Subramaniam, and Alessandro Senes. 2014. "A Frequent, GxxxG-Mediated, Transmembrane Association Motif Is Optimized for the Formation of Interhelical Cα-H Hydrogen Bonds." Proceedings of the National Academy of Sciences of the United States of America 111 (10): E888-895. https://doi.org/10.1073/pnas.1319944111.
Pan, Liqiang, Tian-Min Fu, Wenbin Zhao, Linlin Zhao, Wen Chen, Chixiao Qiu, Wenhui Liu, et al. 2019. "Higher-Order Clustering of the Transmembrane Anchor of DR5 Drives Signaling." Cell 176 (6): 1477-1489.e14. https://doi.org/10.1016/j.cell.2019.02.001.
Russ, William P, and Donald M Engelman. 2000. "The GxxxG Motif: A Framework for Transmembrane Helix-Helix Association." Journal of Molecular Biology 296 (3): 911-19. https://doi.org/10.1006/jmbi.1999.3489.

## Claims

1. A chimeric antigen receptor (CAR) comprising a hinge domain and/or a transmembrane domain derived from a mammalian DR5 receptor.

2. The chimeric antigen receptor of claim 1 comprising:
(i) an antigen-binding domain which binds specifically to a target antigen or a selected cell type expressing the target antigen,
(ii) a hinge domain derived from the ectodomain of a mammalian DR5 receptor,
(iii) a transmembrane domain derived from a mammalian DR5 receptor,
(iv) a co-stimulatory domain, and
(v) an activating domain,
and wherein components (i), to (v) are preferably comprised in N- to C-terminal order.

3. The chimeric antigen receptor of any one of claims 1 or 2,
wherein the antigen-binding domain (i) comprises a functional binding fragment of a natural binding protein, in particular a receptor or an antibody, preferably a single-chain variable fragment (scFv) of an antibody; or a short synthetic protein that binds to the target antigen, preferably a DARPIN or a designed protein binder domain.

4. The chimeric antigen receptor of any one of claims 1 to 3,
wherein the hinge (ii) and transmembrane domain (iii) comprises the hinge and transmembrane region of a mammalian death receptor 5 (DR5), preferably human DR5, or functional fragments thereof having at least 75% identical residues to human DR5, and
wherein the components (i) and (ii) are optionally joined by a short flexible peptide linker.

5. The chimeric antigen receptor of any one of claims 1 to 4,
wherein the co-stimulatory domain (iv) preferably comprises a mammalian 41BB or a CD28 domain, or a functional variant thereof, and the activating domain (v) preferably comprises a mammalian CD3ζ domain, or a functional variant thereof; and
wherein components (iv) and (v) are optionally joined by short peptide linkers.

6. The chimeric antigen receptor of any one of claims 1 to 5,
wherein the co-stimulatory domain or functional variant of (iv) has at least 75% identical residues to the respective human sequence of SEQ ID NO: 39 or 64, and/or
wherein the activating domain or functional variant of (v) has at least 75% identical residues to the human sequence of SEQ ID NO: 40.

7. The chimeric antigen receptor of any one of claims 1 to 6,
wherein the antigen-binding domain (i) specifically binds an antigen on the surface of a pathologic cell, preferably a tumor associated antigen, optionally selected from CD19, BCMA, or ErbB2.

8. The chimeric antigen receptor of any one of claims 1 to 7, wherein the DR5 receptor derived hinge domain (ii) comprises the amino acid amino acid sequence of SEQ ID NO: 1 or a functional variant thereof having at least 75% sequence identity.

9. The chimeric antigen receptor of any one of claims 1 to 8, wherein the DR5 receptor derived transmembrane domain (iii) comprises the amino acid amino acid sequence of SEQ ID NO: 3 or a functional variant thereof having at least 75% sequence identity.

10. The chimeric antigen receptor of any one of claims 1 to 9, wherein the chimeric antigen receptor comprises the amino acid sequences of SEQ ID NOS: 5-10 or functional variants thereof having at least 75% sequence identity, and optionally variants thereof lacking the Myc tag.

11. A nucleic acid, or a plurality of nucleic acids, encoding a CAR of any one of claims 1 to 10, preferably selected from:
**(A)** nucleic acids comprising a sequence encoding SEQ ID NO: 1 and/or SEQ ID NO: 3, or
**(B)** a nucleotide sequence encoding any one of SEQ ID NOs:5-10,
or functional variants thereof having at least 75% sequence identity thereto.

12. A recombinant expression vector comprising a nucleic acid or a plurality of nucleic acids according to claim 11, wherein the expression vector is preferably a viral vector, a transposon or a mRNA.

13. An isolated mammalian cell comprising a CAR according to any one of claims 1 to 10, an expression vector according to claim 12, or a nucleic acid or a plurality of nucleic acids according to claim 11,
wherein the cell is preferably selected from the group consisting of an αβ T cell, a γδ T cell, a natural killer (NK) cell, a natural killer T (NKT) cell, a cytotoxic T lymphocyte (CTL), a regulatory T cell, macrophage or combinations thereof.

14. A pharmaceutical composition comprising a CAR according to any one of claims 1-10, a nucleic acid or a plurality of nucleic acids according to claim 11, a vector according to claim 12, or a cell or population thereof according to claim 13, and at least one component selected from: pharmaceutically acceptable carrier, diluent, adjuvant, and excipient.

15. A CAR according to any one of claims 1-10, a nucleic acid or a plurality of nucleic acids according to claim 11, a vector according to claim 12, a cell or population thereof according to claim 13, and/or a pharmaceutical composition of claim 14, for use in treating, preventing and/or ameliorating a condition or disorder associated with the expression of an antigen, preferably a tumor antigen.
